(19) Europäisches Patentamt
European Patent Office
Office européen des brevets

(11) Veröffentlichungsnummer: **0 032 387**
**B1**

(12) # EUROPÄISCHE PATENTSCHRIFT

(45) Veröffentlichungstag der Patentschrift: **02.11.83**

(51) Int. Cl.³: **C 07 C 103/76**, C 07 C 103/24, A 61 K 49/04

(21) Anmeldenummer: **81100106.4**

(22) Anmeldetag: **09.01.81**

(54) **Nichtionische 5-C-substituierte 2,4,6-Trijodisophthalsäure-Derivate, Verfahren zu ihrer Herstellung und diese enthaltende Röntgenkontrastmittel.**

(30) Priorität: **11.01.80 DE 3001292**

(43) Veröffentlichungstag der Anmeldung: **22.07.81 Patentblatt 81/29**

(45) Bekanntmachung des Hinweises auf die Patenterteilung: **02.11.83 Patentblatt 83/44**

(84) Benannte Vertragsstaaten: **AT BE CH DE FR GB IT LI LU NL SE**

(56) Entgegenhaltungen:
**DE - A - 2 031 724**
**FR - A - 2 354 316**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Gries, Heinz, Dr., Helmstädter Strasse 19, D-1000 Berlin 31 (DE)**
Erfinder: **Pfeiffer, Heinrich, Dr., Pulfrichzeile 9, D-1000 Berlin 20 (DE)**
Erfinder: **Speck, Ulrich, Dr., Benediktiner Strasse 50, D-1000 Berlin 28 (DE)**
Erfinder: **Mützel, Wolfgang, Dr., Weddigenweg 74, D-1000 Berlin 45 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 032 387 B1

# Nichtionische 5-C-substituierte 2,4,6-Trijodisophthalsäure-Derivate, Verfahren zu ihrer Herstellung und diese enthaltende Röntgenkontrastmittel

Die Erfindung betrifft neue, nichtionische, in 5-Stellung C-substituierte 2,4,6-Trijod-isophthalsäure-Derivate der allgemeinen Formel I

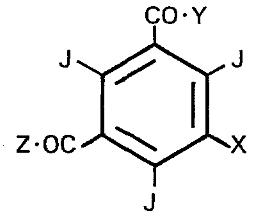

(I),

worin

X den Rest $-CO \cdot N \cdot R_1R_2$, $-COOR_3$, $-CH_2 \cdot NH \cdot R_7$ oder $-CH_2OH$,

Y den Rest $-N \cdot R_1R_2$ oder $-OR_3$,

Z den Rest $-N \cdot R_1R_2$, $-NH \cdot R_7$, $-NH \cdot$Zuckerrest oder $-OH_3$, wobei $-NR_1R_2$ in X, Y und Z gleich oder verschieden sind,

$R_1$ und $R_2$ gleich oder verschieden sind und ein Wasserstoffatom oder einen gegebenenfalls mono- oder polyhydroxylierten gerad- oder verzweigtkettigen Alkylrest,

$R_3$ einen niederen, gegebenenfalls hydroxylierten Alkylrest und

$R_7$ den Acylrest einer gegebenenfalls hydroxylierten niederen aliphatischen Carbonsäure, wobei anwesende OH-Gruppen auch funktionell abgewandelt sein können,

bedeuten, ein Verfahren zu deren Herstellung und neue Röntgenkontrastmittel, die Verbindungen der Formel I als schattengebende Substanz enthalten.

Die unsubstituierten Alkylgruppen $R_1$ und $R_2$, die gerad- oder verzweigtkettig sein können, enthalten 1-6, vorzugsweise 1-4 und insbesondere 1-2 Kohlenstoffatome. Beispielsweise genannt seien insbesondere der Methyl-, Äthyl- und Propylrest. Bevorzugt ist der Methylrest.

Ist der Alkylrest ein Mono- oder Polyhydroxyalkylrest, kann er gerad- oder verzweigtkettig sein. Bevorzugt geeignet sind Alkylreste mit 2-8, vorzugsweise mit 2-4 Kohlenstoffatomen. Die Hydroxylgruppen im Alkylrest können als primäre und/oder sekundäre und/oder tertiäre Hydroxylgruppen vorliegen. Der Alkylrest kann 1-5, vorzugsweise 1-3 Hydroxylgruppen enthalten. Beispielsweise genannt seien der Trishydroxymethyl-methyl-, Hydroxyäthyl-, insbesondere der 1,3- und 2,3-Dihydroxypropyl-, und der 2,3-Dihydroxy-1-hydroxymethyl-propyl-rest.

Der Rest $R_3$ ist ein niederer Alkylrest mit 1-4 Kohlenstoffatomen, vorzugsweise 1-2 Kohlenstoffatomen. Bevorzugt ist der Methylrest. Ist der Rest $R_3$ hydroxyliert, enthält er vorzugsweise 2-4 C-Atome im Alkylrest und trägt 1-3 Hydroxylgruppen, vorzugsweise eine Hydroxylgruppe. Als hydroxylierte Alkylreste $R_3$ seien beispielsweise der Dihydroxypropylrest und vorzugsweise der Hydroxyäthyl-, Dihydroxyäthyl-, Trihydroxypropyl- und Hydroxymethylrest genannt.

Bedeutet bzw. enthält der Substituent Z und/oder X den Rest $-NH-R_7$, so leitet sich der Acylrest $R_7$ von einer aliphatischen Carbonsäure mit 2-5 Kohlenstoffatomen ab. Geeignet sind insbesondere aliphatische Carbonsäurereste mit 2-4 Kohlenstoffatomen wie beispielsweise der Propionylrest und vorzugsweise der Acetylrest.

Bevorzugt geeignet sind Acylreste $R_7$, die durch 1-5, vorzugsweise 1-3 Hydroxylgruppen substituiert sind. Beispielsweise genannt seien der Hydroxyacetyl-, der 2-Hydroxy-propionyl- und der 2,3-Dihydroxypropionylrest.

Liegen die Hydroxygruppen im Acylrest $R_7$ in funktionell abgewandelter Form vor, so liegen sie vorzugsweise als Äthergruppen vor. Beispielsweise genannt sei die Methoxyacetylgruppe.

Seit Einführung der trijodierten Benzoesäurederivate als schattengebende Substanzen in Röntgenkontrastmitteln für die Darstellung von Blutgefässen, ableitenden Harnwegen und anderen Körperhöhlen und Geweben ist eine Vielzahl von Derivaten synthetisiert, geprüft und zum Teil auch praktisch angewandt worden, sowohl in ionischen Kontrastmitteln in Form ihrer Salze als auch in nicht-ionischen Kontrastmitteln.

Dabei erkannte man, dass der unphysiologisch hohe osmotische Druck der Salzzubereitungen für eine Reihe von Unverträglichkeitserscheinungen verantwortlich ist, wodurch die Indikationsbreite dieser Zubereitungen begrenzt wird. Dies hat zur Entwicklung von nicht-ionischen Jodverbindungen hoher Wasserlöslichkeit geführt, deren osmotischer Druck erheblich niedriger liegt.

Als erste, gut verträgliche, lösliche und für die praktische Radiologie geeignete nicht-ionische schattengebende Substanz ist das Metrizamid (DOS 2 031 724) zu nennen. Beim Metrizamid wird die Löslichkeit durch eine Amidbindung des trijodierten Aromaten mit Glucosamin, beim Joglumid (DOS 2 456 685) durch eine Amidbindung des trijodierten Aromaten mit Gluconsäure erzielt. Verbindungen mit derartigen Seitenketten sind schwierig herstellbar, nicht stabil genug, um in der Hitze sterilisiert zu werden und auch nicht ausreichend lagerfähig. Für den praktischen Gebrauch in Röntgenkontrastmitteln ist dies als schwerwiegender Nachteil anzusehen.

Nahezu alle bisher beschriebenen nicht-ionischen Verbindungen leiten sich von den beiden Grundstrukturen Trijoddiaminbenzoesäure und Trijodaminoisophthalsäure ab.

Die Derivate beider Grundkörper entsprechen den immer höheren Anforderungen an ein ideales Röntgenkontrastmittel nicht. Die wichtigsten Anforderungen sind hohe Kontrastdichte, chemische Stabilität und möglichst völlige Untoxizität des Wirkstoffs, niedrige Viskosität der flüssigen Zubereitung und an die Applikationsform angepasste pharmakodynamische Eigenschaften. Das «ideale Kontrastmittel» sollte alle diese Anforderungen in sich vereinigen. Andererseits ist bekannt, dass durch die Anforderungen bezüglich Kontrastdichte, Stabilität und Viskosität die Variationsmöglichkeiten der beiden genannten Grundstrukturen erheblich eingeschränkt sind, insbesondere auch im Hinblick darauf, dass für den praktischen Gebrauch im allgemeinen Substanzen mit hohem Jodgehalt in Frage kommen. Da die Synthesemöglichkeiten inzwischen weitgehend erschöpft sind, ist die Einführung einer neuen Grundstruktur von besonderem Wert.

Die relativ gute Verträglichkeit der heute gebräuchlichen Röntgenkontrastmittel wird dadurch erreicht, dass die an und für sich lipophilen und toxischen Grundkörper durch stark hydrophile Substituenten entgiftet werden.

Der vorliegenden Erfindung lag die Aufgabe zugrunde, neue Grundstrukturen zu entwickeln, die bereits selbst möglichst hydrophil und untoxisch sind und daraus neue und bessere Kontrastmittel herzustellen.

Die vorliegende Erfindung umfasst nicht-ionische Röntgenkontrastmittel auf Basis schattengebender Substanzen mit neuen Grundstrukturen. Diese neuen erfindungsgemässen schattengebenden Substanzen sind durch eine Reihe von Vorteilen ausgezeichnet: diese neuen Verbindungen leiten sich ab von trijodierten Aromaten als Grundkörper, die selbst schon hydrophil und relativ untoxisch sind. Die Einführung allzu umfangreicher hydrophiler Substituenten zur Verminderung der Chemotoxizität war somit entbehrlich geworden, wodurch die erfindungsgemässen Verbindungen einen erwünscht hohen Jodgehalt besitzen. Sie sind ausgezeichnet durch eine hohe chemische Stabilität, insbesondere auch unter den Bedingungen der Hitzesterilisation.

Lösungen der erfindungsgemässen Verbindungen der Formel I können somit in gebräuchlicher Weise auch durch Erhitzen auf 120°C bei physiologischem $p_H$-Wert sterilisiert werden. Die Lösungen haben auch bei hoher Jodkonzentration verglichen mit den zur Zeit gebräuchlichen ionischen Röntgenkontrastmitteln einen niedrigen osmotischen Druck, was insbesondere für eine gute lokale Verträglichkeit Voraussetzung ist.

Die erfindungsgemässen Substanzen zeigen in verschiedenen Testen bei unterschiedlichen Tierspezies sehr gute allgemeine und ausgezeichnete lokale Verträglichkeit, eine sehr gute Herzkreislaufverträglichkeit und nur geringe Neurotoxizität. Darüber hinaus zeigen die erfindungsgemässen Substanzen im in-Vitro-Test eine nur äusserst geringe Interaktion mit Proteinen und nur sehr geringe membranschädigende Wirkung.

Schliesslich zeigen die neuen Verbindungen nur geringe epileptogene Wirkung nach subarachnoidaler Verabreichung.

Die gut wasserlöslichen Verbindungen sind für alle Anwendungen geeignet, in denen jodhaltige, nierengängige Kontrastmittel eingesetzt werden, wie z.B. Angiographie, Urographie, Computertomographie, Magen-Darmdarstellung, Arthographie und Myelographie. Bevorzugt werden die erfindungsgemässen Verbindungen angewandt auf den Gebieten Angiographie, Myelographie und bei solchen Indikationen, in denen das Röntgenkontrastmittel nicht wie nach i.v.-Indikation sehr rasch verdünnt wird, so dass die lokale Verträglichkeit eine bedeutende Rolle spielt.

Die schwer wasserlöslichen Verbindungen sind wegen ihres dennoch ausserordentlich hydrophilen Charakters ebenfalls gut verträglich. Ihre lokale Verträglichkeit ist jedenfalls deutlich besser als die der bisher experimentell und klinisch verwendeten Ester von ionischen Kontrastmitteln wie dem Äthylester der Iothalamsäure, des Iodipamids und der Iopodinsäure. Als Anwendungsgebiet dieser Verbindungen kommen alle in der Literatur beschriebenen Möglichkeiten zur Verwendung von partikelhaltigen Kontrastmitteln in Frage, wie Angiographie, Computertomographie, direkte und indirekte Lymphographie, Magen-Darmdarstellung, Bronchographie und Darstellung anderer Körperhöhlen. Bevorzugt wird in solchen Fällen die Anwendung als Pulver oder Mikrokristallsuspensionen, die gegebenenfalls geeignete Stabilisatoren wie Gelatine, Humanalbumin, Dextran, Polyvinylpyrrolidon u.ä. enthalten. Die so verabreichten, wenig wasserlöslichen neuen Kontrastmittel werden im Organismus langsam aufgelöst und überwiegend renal ausgeschieden.

In der nachfolgenden Tabelle seien einige der oben erwähnten vorteilhaften Eigenschaften der neuen nicht-ionischen Röntgenkontrastmittel am Beispiel der schattengebenden Substanzen 2,4,6-Trijodbenzol-1,3,5-tricarbonsäure-tris-(2,3-dihydroxypropyl-N-methyl)-triamid (B), 2,4,6-Trijodbenzol-1,3,5-tricarbonsäure-tris-(bis-2-hydroxyäthyl)-triamid (C), 2,4,6-Trijodbenzol-1,3,5-tricarbonsäure-bis-(2,3-dihydroxypropyl-N-methyl)-triamid (D) und 2,4,6-Trijodbenzol-1,3,5-tricarbonsäure-tris-(2,3,4,5,6-pentahydroxyhexyl-N-methyl-triamid (E),2,4,6-Trijodbenzol-1,3,5-tricarbonsäure-[(N,N-dimethyl)-bis-(2,3-dihydroxypropyl)]-triamid (F) und 5-N-Methyl-carbamoyl-2,4,6-trijodisophthalsäure-bis-(2,3-dihydroxy-1-hydroxymethylpropyl)-diamid (G) im Vergleich zu dem bekannten Matrizamid (A) gegenübergestellt.

Tabelle 1

Eigenschaften neuer nichtionischer Röntgenkontrastmittel im Vergleich zu Metrizamid

| Substanz | Jodgehalt | stabil bei Hitzesterilisation | Verteilungskoeffizient Butanol/Puffer pH 7.6 | Erythrozytenschädigung | $DL_{50}$ Maus i.v.*) (g Jod/kg) | $DL_{50}$ Ratte i.v.*) (g Jod/kg) |
|---|---|---|---|---|---|---|
| A | 48 | nein | 0,259 | 10 | 12 | 11 |
| B | 45 | ja | 0,064 | 2,1 | 16 | 11 |
| C | 45 | ja | 0,131 | 1,8 | | 12 |
| D | 50 | ja | 0,074 | 4,2 | | |
| E | 34 | ja | <0,001 | <0,3 | | |
| F | 50 | ja | 0,171 | 2,7 | 14 | 11 |
| G | 47 | ja | 0,065 | 2,8 | | 19 |

*) Injektionsgeschwindigkeit 2 ml/min; Konzentration 300 mg/ml; Beobachtungszeit 24h

Der Verteilungskoeffizient einer Substanz gestattet eine Aussage über ihre Hydrophilität und damit über ihre Verträglichkeit; je niedriger der Wert des Verteilungskoeffizienten, um so besser ist die Verträglichkeit der getesteten Substanz.

Die Verformung von Erythrozyten (Erythrozytenschädigung) durch Röntgenkontrastmittel kann als Mass für die membranschädigende Wirkung der Verbindung gewertet werden. Gemessen wurde hier die Bildung von Echinocyten an Hundeerythrocyten, wobei die Wirkung von Metrizamid = 10 gesetzt wurde. Eine kleinere Zahl bedeutet eine entsprechend bessere Verträglichkeit.

Die Erfindung bestrifft somit auch neue Röntgenkontrastmittel auf Basis von Verbindungen der allgemeinen Formel (I).

Die Herstellung der neuen Röntgenkontrastmittel auf Basis der erfindungsgemässen Verbindungen der allgemeinen Formel (I) erfolgt in an sich bekannter Weise, z.B. dadurch, dass man die schattengebende Substanz mit den in der Galenik üblichen Zusätzen, z.B. Stabilisatoren wie Natriumedetat, Calcium-di-natriumedetat, physiologisch verträglichen Puffern, Natriumchlorid u.ä., in eine für die intravenöse Applikation geeignete Form bringt. Die Konzentration der neuen Röntgenkontrastmittel im wässrigen Medium richtet sich ganz nach der röntgendiagnostischen Methode. Die bevorzugten Konzentrationen und Dosierungen der neuen Verbindungen bewegen sich in den Bereichen von 50-400 mg J/ml für die Konzentration und 5-500 ml für die Dosierung. Besonders bevorzugt sind Konzentrationen zwischen 100-400 mg J/ml.

Die Erfindung betrifft ferner ein Verfahren zur Herstellung von Verbindungen der allgemeinen Formel I, dadurch gekennzeichnet, dass man in an sich bekannter Weise

a) eine Verbindung der allgemeinen Formel II

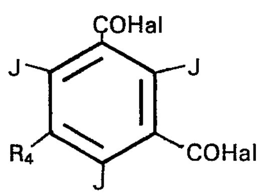

(II),

worin

Hal ein Halogenatom, vorzugsweise ein Cl-Atom, und

$R_4$ die Gruppe -COHal, $-CON<^{R_1}_{R_2}$, -CONH-$R_7$, $-CH_2 \cdot NH \cdot R_7$ oder $-CH_2OH$

(worin Hal, $R_1$, $R_2$ und $R_7$ die oben angegebene Bedeutung haben) mit einer Base der allgemeinen Formel IIIA

$$HN<^{R'_1}_{R'_2} \qquad \text{(IIIA)},$$

($R'_1$ und $R'_2$ das gleiche wie $R_1$ und $R_2$ aber nicht gleichzeitig Wasserstoff bedeuten) oder stufenweise die 1-ständige -COHal-Gruppe mit der Base IIIA und die 3-ständige -COHal-Gruppe mit einer Base IIIB der allgemeinen Formel

$$HN<^{R''_1}_{R''_2} \qquad \text{(IIIB)},$$

(worin $R''_1$ und $R''_2$ das gleiche wie $R_1$ und $R_2$ aber nicht gleichzeitig Wasserstoff bedeuten und verschieden von $R'_1$ und/oder $R'_2$ sind) umsetzt, oder

b) eine Verbindung der allgemeinen Formel IV

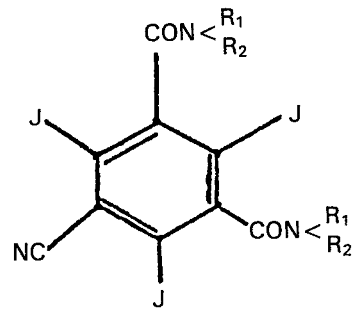

(IV),

worin $R_1$ und $R_2$ die obengenannte Bedeutung hat, mit dem reaktionsfähigen Derivat einer aliphatischen Carbonsäure $R_7$-OH umsetzt, oder in wässriger Lösung mit Alkali behandelt und die so erhaltene 5-Carbamoyl-Verbindung gewünschtenfalls anschliessend in wässriger Lösung und Gegenwart einer starken Säure diazotiert und die gebildete 5-Carboxylgruppe mit einem 2-Amino-Zucker oder mit der Base $HNR'_1R'_2$ ($R'_1$ und $R'_2$ haben die oben angegebene Bedeutung) amidiert oder mit einem Alkohol $R_3OH$ verestert oder

c) eine Verbindung der allgemeinen Formel V

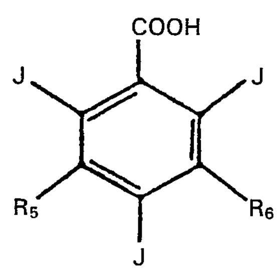

(V),

worin

$R_5$ die Reste $-CH_2OH$, $-CON<^{R_1}_{R_2}$, $-CH_2 \cdot NH \cdot R_7$, $-CONHR_7$ oder -COOH und

$R_6$ die Reste $-CON<^{R_1}_{R_2}$ oder -COOH,

mit einem Alkohol $R_3OH$ ($R_3$ hat die oben angegebene Bedeutung) verestert,

bedeuten,

und gewünschtenfalls anschliessend Wasserstoff enthaltende Aminogruppen mit einem reaktionsfähigen Derivat einer aliphatischen Carbonsäure $R_7$-OH ($R_7$ hat die obengenannte Bedeutung) acyliert und/oder mit einem $R'_1$-enthaltenden Alkylierungsmittel N-alkyliert und/oder geschützte Hydroxylgruppen in Freiheit setzt.

Die im Verlauf des Verfahrens zur Herstellung der erfindungsgemässen Verbindungen der Formel II notwendigen Amidierungsreaktionen erfolgen nach an sich bekannten Methoden.

Der Rest Hal im Ausgangsprodukt der allgemeinen Formel II bedeutet ein Halogenatom, z.B. Jod, Brom oder insbesondere Chlor.

Für die Amidierungsreaktion können in den Substituenten $R'_1$, $R'_2$, $R''_1$ und $R''_2$ anwesende Hydroxylgruppen in freier oder geschützter Form vorliegen. Sollen diese Hydroxylgruppen in geschützter Form vorliegen, kommen alle üblichen Hydroxylschutzgruppen in Frage, die bekanntermassen für einen intermediären Hydroxylgruppenschutz geeignet sind, d.h. die sich leicht einführen und sich später unter Rückbildung der letztlich gewünschten freien Hydroxylgruppe auch wieder leicht abspalten lassen. Bevorzugt ist der Schutz durch Acylierung, insbesondere Acetylierung oder durch Acetalisierung mit z.B. Acetaldehyd, oder durch Ketalisierung mit z.B. Aceton oder 2,2-Dimethoxypropan. Geeignete Schutzgruppen sind auch Äthergruppen wie z.B. Benzyl-, Di- und Triphenylmethyl-Äthergruppen.

Die Amidierung der beiden 1- und 3-ständigen CO·Hal-Gruppen kann in einem Reaktionsschritt oder auch stufenweise erfolgen. Sind die beiden 1- und 3-ständigen Amidreste im letztlich gewünschten Verfahrensprodukt bezüglich der N-Substituenten $R_1$ und $R_2$ gleich, erfolgt die Amidierung vorzugsweise in einem Reaktionsschritt. Unterscheiden sich jedoch diese beiden Amidgruppen bezüglich der N-Substituenten $R_1$ und $R_2$, so erfolgt die Amidierung vorzugsweise stufenweise.

Die beiden Amidierungsreaktionen erfolgen in einem geeigneten Lösungsmittel bei 0-100°C, vorzugsweise bei 20-80°C. Geeignete Lösungsmittel sind u.a. polare Lösungsmittel. Beispielsweise genannt seien Wasser, Dioxan, Tetrahydrofuran, Dimethylformamid, Dimethylacetamid, Hexametapol u.ä. und deren Gemische. Da die Amidierungsreaktion exotherm verläuft, ist es gegebenenfalls zweckmässig, das Reaktionsgemisch zu kühlen. Da bei der Amidierungsreaktion Halogenwasserstoff, z.B. Chlorwasserstoff, frei wird, der zwecks Neutralisation gebunden werden muss, benötigt man pro Säurechloridgruppe zwei Äquivalente Base, zweckmässigerweise im Überschuss von ca. 10%.

Zur Herstellung von Endprodukten, in denen die einzuführenden Amidgruppen gleich sind, wird das gelöste Ausgangsprodukt II umgesetzt mit 4 Äquivalenten der Base IIIA oder mit 2 Äquivalenten der Base IIIA in Gegenwart von 2 Äquivalenten einer vorzugsweise tertiären Base, die dann als Protonenakzeptor dient.

Zur Herstellung von Endprodukten, in denen die einzuführenden Amidgruppen verschieden sind, wird das gelöste Ausgangsprodukt zunächst mit 2 Äquivalenten der Base IIIA oder mit einem Äquivalent der Base IIIA in Gegenwart einer vorzugsweise tertiären Base umgesetzt.

Das Monoamid wird zur Vermeidung von Nebenreaktionen bei der Weiterverarbeitung zweckmässigerweise in üblicher Weise isoliert und in zweiter Stufe in analoger Weise mit der Base IIIB zum Diamid umgesetzt.

Erfolgt die erste Amidierungsstufe mit Base IIIA in Gegenwart einer vorzugsweise tertiären Base, kann die zweite Amidierungsstufe mit Base IIIB gegebenenfalls auch ohne Isolierung des primär entstandenen Monoamids im Eintopfverfahren durchgeführt werden.

Ist im Ausgangsprodukt der allgemeinen Formel II auch der Substituent $R_4$ eine -CO·Hal-Gruppe, erhält man bei Amidierung mit 6 Äquivalenten Base IIIA bzw. mit 3 Äquivalenten Base IIIA in Gegenwart von 3 Äquivalenten einer vorzugsweise tertiären Base das entsprechende 1,3,5-Trisamid der 2,4,6-Trijodtrimesinsäure, in der die drei Amidgruppen gleich sind.

Auch in diesem Fall ist es grundsätzlich möglich, die Amidierung der drei Carboxylgruppen stufenweise vorzunehmen. Wird in erster Stufe mit einer Base IIIA, in zweiter Stufe mit einer Base IIIB und in dritter Stufe mit einer Base HN $R'''_1$ $R'''_2$ (IIIC; $R'''_1$ und $R'''_2$ bedeuten das gleiche wie $R_1$ und $R_2$ aber nicht gleichzeitig Wasserstoff und ist verschieden von $R'_1$, $R'_2$, $R''_1$ und $R''_2$) amidiert, lassen sich also auch 1,3,5-Trisamide der allgemeinen Formel I herstellen, in denen die drei Amid-Gruppen in bezug auf $R_1$ und $R_2$ unterschiedlich N-substituiert sind.

Zur Bindung des bei der Amidierung entstehenden Chlorwasserstoffs verwendet man vorteilhaft tertiäre Basen, wie z.B. Triäthylamin, Tributylamin oder Pyridin. Anwendbar sind aber auch anorganische Protonenakzeptoren wie z.B. Calciumcarbonat.

Die im Reaktionsverlauf anfallenden organischen Salze werden in bekannter Weise abgetrennt, vorteilhaft z.B. mit Hilfe üblicher Ionenaustauscher wie beispielsweise Amberlite JR 120 oder Säulen oder durch Filtration über bekannte Adsorberharze wie z.B. Amberlite XAD-2 und 4.

Macht es der Reaktionsverlauf erforderlich, in den Substituenten $R_1$ und/oder $R_2$ und/oder $R_3$ und/oder $R_7$ anwesende freie Hydroxylgruppen intermediär zu schützen, so erfolgt dies nach üblichen Methoden durch leicht reversible Gruppen. Die Einführung solcher Schutzgruppen kann z.B. erfolgen durch Acylierung (z.B. Einführung eines vorzugsweisen Acetylrestes oder Benzoylrestes) oder durch Verätherung (z.B. Einführung des Triphenylmethylrestes).

Der Hydroxylgruppenschutz kann auch durch Ketalisierung oder Acetalisierung, z.B. mittels Acetaldehyd, Aceton oder Dihydropyran, erreicht werden.

Die spätere Abspaltung der intermediär eingeführten Schutzgruppen unter Freisetzung der letztlich gewünschten Hydroxylgruppen erfolgt ebenfalls nach Methoden, die dem Fachmann allgemein geläufig sind. So kann die Abspaltung der Schutzgruppen ohne besondere Reaktionsstufe mit der Aufarbeitung und Isolierung der Umsetzungsprodukte erfolgen. Sie kann aber auch in üblicher Weise in einer getrennten Reaktionsstufe durchgeführt werden. Acylschutzgruppen können beispielsweise durch alkalische und Acetal-, Ketal- oder Ätherschutzgruppen durch saure Hydrolyse abgespalten werden.

Die Überführung der Cyano-Gruppe in die N-acylierte Amido-Gruppe -CONH·$R_7$ erfolgt durch Anlagerung einer aliphatischen Carbonsäure $R_7COOH$ an die CN-Dreifachbindung nach dem Fachmann bekannten Methoden. Zur praktischen Durchführung wird die Säure $R_7COOH$ zweckmässigerweise in Form eines reaktiven Derivats, vorzugsweise als Anhydrid, zur Einwirkung gebracht. Die Umsetzung erfolgt in Gegenwart eines geeigneten sauren Kataly-

satorѕ, wie z.B. Perchlorsäure oder Schwefelsäure, Phosphorsäure und ähnliche. In der Regel dient das eingesetzte Säureanhydrid auch gleichzeitig als Lösungsmittel, was nicht ausschliesst, dass man dem Reaktionsgemisch einen geeigneten Lösungsvermittler wie Dioxan zusetzt. Die Umsetzung erfolgt bei Raumtemperatur oder erhöhter Temperatur. Erfolgt die Umsetzung bei erhöhtr Temperatur, ist der bevorzugte Temperaturbereich 40-110°C.

Die Überführung der Cyanogruppe im Ausgangsprodukt der Formel IV in die Carbamoylgruppe erfolgt ebenfalls nach Methoden wie sie dem Fachmann geläufig sind. Zweckmässigerweise wird das Ausgangsprodukt in Wasser in Gegenwart von überschüssigem Alkali bei Raumtemperatur oder erhöhter Temperatur, z.B. bei 0-80°C, hydrolysiert. Als Alkali dient insbesondere Kalium- oder Natriumhydroxid, das man dem Reaktionsgemisch in fester Form oder als z.B. 2n Alkalihydroxidlösung zugeben kann. Gewünschtenfalls kann man dem wässrigen Reaktionsgemisch auch einen organischen Lösungsvermittler, wie beispielsweise Methanol, Dioxan, Tetrahydrofuran oder ähnliche organische Lösungsmittel zusetzen.

Die partielle Verseifung der Cyanogruppe zur Carbamoylgruppe kann auch in saurem $p_H$-Bereich durchgeführt werden, z.B. in konzentrierter Schwefelsäure bei erhöhter Temperatur, z.B. 50-90°C.

Die gewünschtenfalls anschliessende Diazotierung der Carbamoylgruppe zur Carboxylgruppe erfolgt in an sich bekannter Weise mit den dazu üblichen Reagenzien wie beispielsweise Nitrosylchlorid, Nitrosylschwefelsäure oder Natrium- oder Kaliumnitrit in Gegenwart einer Säure, wie beispielsweise Chlorwasserstoffsäure, Schwefelsäure, u.a. Zur praktischen Durchführung wird die zu diazotierende Substanz z.B. in einem Gemisch Wasser/konz. Salzsäure suspendiert und langsam mit einer wässrigen Natriumnitritlösung versetzt. Die Umsetzung erfolgt bei Raumtemperatur oder zweckmässigerweise erhöhter Temperatur, vorzugsweise bei 40-100°C.

Gleich gut kann die Diazotierung auch in der Weise durchgeführt werden, dass man die Carbamoylverbindung in einem organischen Lösungsmittel wie z.B. Essigsäure oder Dimethylformamid mit Nitrosylchlorid oder mit Nitrosylschwefelsäure umsetzt.

Soll die freie Carboxylgruppe erfindungsgemäss in die Amidgruppe $-NR_1R_2$ oder -NH·Zuckerrest überführt werden, erfolgt die Amidierung in üblicher Weise, z.B. indem man gegebenenfalls in Gegenwart eines geeigneten Lösungsmittels, z.B. Dimethylformamid, Toluol, Acetonitril u.ä. die Carboxylgruppe zunächst mit z.B. Thionylchlorid, Phosphorpentachlorid, Phosgen oder 1,1-Dichlormethylmethyläther in die Säurechloridgruppe überführt und diese wie oben näher erläutert mit $HN\,R'_1R'_2$ (IIIA) oder einem Aminozucker umsetzt.

Als Aminozucker, in dem die Aminogruppe vorzugsweise in 2-Stellung gebunden ist, kommen vor allem solche mit 4-6 Kohlenstoffatomen im Zuckerrest in Frage. Bevorzugt geeignet ist die 2-Aminoglucose.

Soll die Carboxylgruppe letztlich als Estergruppe $-COOR_3$ vorliegen, erfolgt die Veresterung der freien Carboxylgruppe nach an sich bekannten Methoden.

Enthält der Esterrest $-COOR_3$ Hydroxylgruppen im Alkylrest, führt man einen solchen Esterrest zweckmässigerweise so ein, dass man das Alkali-Salz der Säure, vorzugsweise das Natriumsalz, in einem geeigneten Lösungsmittel, vorzugsweise Dimethylformamid oder auch Dimethylacetamid, mit dem entsprechenden Alkylhalogenid, vorzugsweise Chlorid umsetzt.

Neben dieser im Rahmen der vorliegenden Erfindung bevorzugten Veresterungsmethode kann die Veresterung der freien Carboxylgruppen auch nach anderen dafür gebräuchlichen Methoden vorgenommen werden, insbesondere auch dann, wenn der einzuführende $R_3O$-Rest keine zusätzlichen Hydroxylgruppen im Alkylrest $R_3$ enthält. Beispielsweise genannt sei hier die Veresterung mit entsprechenden Diazoalkanen, z.B. Diazomethan, Diazoäthan oder die Umsetzung der freien Carboxylgruppe mit einem Alkohol $R_3OH$, vorzugsweise in Gegenwart z.B. einer Mineralsäure wie Schwefelsäure.

Die erfindungsgemässen Verbindungen der Formel I können 1-3 veresterte Carboxylgruppen enthalten, wobei bei mehr als einer Estergruppe im Molekül, die Esterreste $-COOR_3$ gleich oder verschieden sein können.

Die Veresterung freier Carboxylgruppen im Ausgangsprodukt IV oder V erfolgt nach dem Fachmann allgemein bekannten Methoden. Als bevorzugte Veresterungsmethode sei beispielsweise die Umsetzung der Carboxylgruppe in Form des Alkalisalzes mit $R_3$-Halogenid — wie oben bereits näher erläutert — genannt.

Zur Herstellung von Verbindungen der Formel I, die nur eine $-COOR_3$-Gruppe enthalten, geht man zweckmässigerweise von Verbindungen der Formel IV aus und überführt die 5-ständige Cyanogruppe gemäss Verfahrensvariante b in die letztlich gewünschte Estergruppe $-COOR_3$.

Sollen die letztlich gewünschten Verbindungen der Formel I zwei oder auch drei $-COOR_3$-Gruppen enthalten, erfolgt deren Herstellung zweckmässigerweise gemäss Verfahrensvariante c, wobei im Ausgangsprodukt der Formel V der Rest $R_5$ und/oder $R_6$ -COOH bedeuten.

Enthalten die so erhaltenen erfindungsgemässen Verbindungen primäre und/oder sekundäre Amidgruppen, können diese gewünschtenfalls nach verschiedenen, dem Fachmann bekannten Methoden noch N-alkyliert und/oder acyliert werden, gegebenenfalls nach intermediärem Schutz freier Hydroxylgruppen.

Die nachträgliche N-Alkylierung führt man z.B. in der Weise durch, dass man auf das entsprechende Säureamid zunächst einen Protonenakzeptor wie Natriumamid, Natriumhydrid oder auch Alkalihydroxid einwirken lässt und dann umsetzt mit einem $R_1$- bzw. $R'_1$-Alkylhalogenid, vorzugsweise als Bromid, oder insbesondere mit einem Di-$R_1$ (bzw. $R'_1$)-sulfat (z.B. Dimethyl- oder Diäthylsulfat). Je nach dem verwendeten Protonenakzeptor erfolgt die Umsetzung in wasserfreiem oder wässrigem Reaktionsmittel bei einer Reaktionstemperatur von ca. Raumtemperatur bis 100°C, vorzugsweise 50-70°C.

Geeignete Lösungsmittel bzw. Lösungsvermittler

sind bekanntermassen Aceton, Dimethylformamid, Dioxan, Tetrahydrofuran u.ä.

Sollen die zunächst erhaltenen erfindungsgemässen Verbindungen der Formel I gewünschtenfalls noch acyliert werden, erfolgt die Acylierung der noch Wasserstoff enthaltenden Amidgruppen ebenfalls nach an sich bekannten Verfahren, indem man z.B. das Amid in einem inerten Lösungsmittel, wie z.B. Pyridin, DMA, DMF u.ä. bei Temperaturen von 0°C bis Raumtemperatur mit einem reaktiven Säurederivat, vorzugsweise mit dem entsprechenden Säurehalogenid, insbesondere Säurechlorid oder aber auch mit einem entsprechenden Säureanhydrid, vorzugsweise in Gegenwart eines sauren Katalysators, wie z.B. $H_2SO_4$, umsetzt.

Die verfahrensgemäss eingesetzten Ausgangsprodukte können nach an sich bekannten Methoden beispielsweise aus den bekannten Verbindungen der allgemeinen Formel

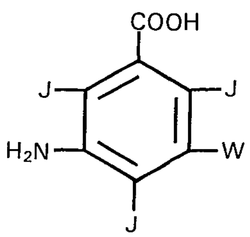

worin W die Reste -COOH, $-CH_2NH \cdot R_7$ oder $-CH_2OH$ bedeuten, hergestellt werden, indem man in beliebiger Reihenfolge die 5-ständige Aminogruppe mittels Sandmeyer-Reaktion durch die Cyanogruppe substituiert und die Substituenten -COOH, W und CN nach Methoden, wie sie dem experimentell arbeitenden Chemiker geläufig sind, in die letzlich gewünschten Reste der erfindungsgemäss eingesetzten Vor- bzw. Ausgangsprodukte überführt, wie mit den nachfolgenden Ausführungen im einzelnen noch erläutert werden soll.

Die Substitution der aromatischen Aminogruppe durch die Cyanogruppe sei am Beispiel der Herstellung von 5-Cyano-2,4,6-trijod-isophthalsäure nochmals im einzelnen näher erläutert:

112 g 5-Amino-2,4,6-trijod-isophthalsäure werden in 1100 ml Wasser suspendiert und durch Zugabe von 10 g Ätznatron in Lösung gebracht. Dann kühlt man die Lösung, die mittels Schwefelsäure auf pH 2,5 eingestellt wurde, auf 0°C ab und tropft unter Kühlung eine Lösung von 20 g Natriumnitrit in 60 ml Wasser zu, wobei die Reaktionstemperatur bei 0 bis 5°C gehalten wird. Dann stellt man den pH-Wert des Reaktionsgemisches durch Zutropfen von verdünnter Schwefelsäure erneut auf 2,5 ein und rührt unter Eiskühlung ein bis zwei Stunden nach. Der dabei entstandene Niederschlag wird unter Kühlung durch langsames Zutropfen verdünnter Natronlauge bei pH 4,5 in Lösung gebracht. Anschliessend wird die neutralisierte Diazoniumsalzlösung in eine 30°C warme Lösung von 99 g Kupfer-I-chlorid und 172 g Kaliumcyanid in 800 ml Wasser eingegossen, wobei starkes Aufschäumen eintritt, und 15 Minuten bei 30°C gerührt. Das Reaktionsgemisch wird dann mit verdünnter Schwefelsäure auf pH 2,8-3 angesäuert, und das ausgefallene Kupfersalz abgesaugt. Die filtrierte Lösung wird nun durch weitere Zugabe von verdünnter Schwefelsäure auf pH 0,5-1 gebracht. Der entstandene Niederschlag wird nach mehrstündigem Rühren im Eisbad abgesaugt, mit Wasser gewaschen und bei 50°C getrocknet. Zur Reinigung suspendiert man das Rohprodukt in 400 ml Wasser, löst durch Zugabe von Natronlauge, behandelt die Lösung 30 Minuten mit 10 g Aktivkohle und versetzt die filtrierte Lösung mit einem Überschuss an Mineralsäure. Nach mehrstündigem Rühren im Eisbad wird der Niederschlag abgesaugt, mit Wasser gewaschen und bei 50°C getrocknet. Man erhält so 89 g (78% der Theorie) 5-Cyano-2,4,6-trijod-isophthalsäure als weisses Pulver mit einem Zersetzungspunkt oberhalb 300°C.

In analoger Weise werden aus den entsprechenden 5-Amino-Verbindungen hergestellt:

5-Cyano-2,4,6-trijod-isophthalsäure-monomethylamid: Fp. oberhalb 300°C (Zersetzung); Ausbeute 72% der Theorie.

5-Cyano-2,4,6-trijod-isophthalsäure-bis-(2-hydroxy-äthyl)-amid: Fp. oberhalb 300°C Zersetzung; Ausbeute 68% der Theorie.

5-Cyano-2,4,6-trijod-isophthalsäure-mono-N(2--hydroxy-äthyl)-amid: Fp. oberhalb 300°C Zersetzung; Ausbeute 95% der Theorie.

5-Cyano-3-acetylaminomethyl-2,4,6-trijodbenzoesäure: Fp. 271°C (Zersetzung); Ausbeute 85% der Theorie.

5-Cyano-2,4,6-trijod-isophthalsäure-mono-dimethylamid: Fp. 240°C (Zersetzung); Ausbeute 85% der Theorie.

5-Cyano-N-(2-hydroxyäthyl)-N-methyl-2,4,6-trijod-isophthalamsäure: Fp. oberhalb 280°C (Zersetzung); Ausbeute 89% der Theorie.

5-Cyano-3-hydroxymethyl-2,4,6-trijodbenzoesäure: Fp. 250-252°C (Zersetzung); Ausbeute 81% der Theorie.

5-Cyano-2,4,6-trijod-isophthalsäure-mono-amid: Fp. oberhalb 300°C Zersetzung; Ausbeute 82% der Theorie.

Zur Herstellung des Ausgangsproduktes der allgemeinen Formel II (Verfahrensvariante a) geht man zweckmässigerweise von 5-Cyano-Vorprodukten aus.

Je nach der letztlich gewünschten Bedeutung des 5-ständigen Substituenten (in Verbindung II = $R_4$) wird die Cyano-Gruppe zunächst in an sich bekannter Weise sauer oder alkalisch verseift, wobei man das entsprechende Amid erhält. Am Beispiel der 5-Cyano-2,4,6-trijod-isophthalsäure sei diese Verseifungsreaktion näher erläutert:

100 g 5-Cyano-2,4,6-trijod-isophthalsäure werden in 400 ml Wasser suspendiert und durch Zugabe von 20 g Natriumhydroxid in Lösung gebracht. Die Lösung wird 3 Stunden auf + 60°C gehalten, anschliessend unter Rühren in 60 ml konzentrierte Salzsäure eingegossen. Nach mehrstündigem Rühren im Eisbad wird der ausgeschiedene Niederschlag abgesaugt, mit wenig eiskaltem Wasser gewaschen und bei 50°C getrocknet. Man erhält 98 g (= 95% der Theorie) 5-Carbamoyl-2,4,6-trijod-isophthalsäure mit einem Zersetzungspunkt oberhalb 280°C als weisses Pulver.

In analoger Weise werden die folgenden entsprechenden Vor- bzw. Ausgangsprodukte hergestellt:

5-Carbamoyl-N-(2-hydroxyäthyl)-2,4,6-trijodisophthalamsäure: Fp. 310-312°C (Zersetzung) Ausbeute 49,7% der Theorie.

5-Carbamoyl-2,4,6-trijod-isophthalsäure-mono-dimethylamid: Fp. 255°C; Ausbeute 85% der Theorie.

5-Carbamoyl-N-(2-hydroxyäthyl)-N-methyl-2,4,6-trijodisophthalamsäure: Fp. 286-288°C; Ausbeute 55,9% der Theorie.

5-Carbamoyl-2,4,6-trijod-isophthalsäure-mono-methyl-mono-amid: gereinigt über das Dimethylaminsalz: Fp. > 300°C; Ausbeute 76,5% der Theorie.

5-Hydroxymethyl-2,4,6-trijodisophthalamsäure: Fp. oberhalb 300°C (Zersetzung); Ausbeute 78% der Theorie.

5-Acetylaminomethyl-2,4,6-trijodisophthalamsäure: Fp. 220-222°C; Ausbeute 75% der Theorie.

5-Carbamoyl-2,4,6-trijod-isophthalsäure-bis-(2-hydroxyäthyl)-diamid: Fp. oberhalb 300°C (Zersetzung); Ausbeute 75% der Theorie.

5-Carbamoyl-2,4,6-trijod-isophthalsäure-mono-(2,3-dihydroxypropyl)-mono-(2,3-dihydroxypropyl-N-methyl)-diamid: Fp. 202°C; Ausbeute 61% der Theorie.

Die so erhaltene 5-Carbamoylgruppe lässt sich in üblicher Weise in die Carboxylgruppe überführen, zweckmässigerweise in wässrig-saurer Lösung mittels eines Diazotierungsreagenzes, z.B. Natriumnitrit, wie bereits oben ausgeführt und am Beispiel der 2,4,6-Trijod-trimesinsäureherstellung nochmals näher erläutert werden soll:

100 g 5-Carbamoyl-2,4,6-trijod-isophthalsäure werden in 2 l halbkonzentrierter Chlorwasserstoffsäure suspendiert und die Suspension unter Rühren auf 90°C erwärmt. Unter die Oberfläche dieser Lösung wird innerhalb von 5 Stunden eine Lösung von 59 g Natriumnitrit in 1 l Wasser zugeleitet, dann wird die Lösung noch 2 Stunden bei 90°C nachgerührt. Anschliessend wird die Lösung im Vakuum zur Trockne eingeengt und der Rückstand 1 Stunde mit 1 l Äther ausgerührt. Man saugt vom ausgeschiedenen Natriumchlorid ab und erhält nach Eindampfen der ähterischen Lösung 98 g (99% der Theorie) 2,4,6-Trijod-1,3,5-tricarbonsäure als weisses Pulver mit einem oberhalb 280°C liegenden Zersetzungspunkt; Ausbeute 99% der Theorie.

In analoger Weise wird aus der entsprechenden 5-Carbamoyl-Verbindung hergestellt:

2,4,6-Trijodbenzol-1,3,5-tricarbonsäure-monomethyl-mono-amid: Fp. > 300°C; Ausbeute 99% der Theorie.

2,4,6-Trijodbenzol-1,3,5-tricarbonsäure-(2-hydroxyäthyl)-mono-amid: Fp. oberhalb 300°C; Ausbeute 63% der Theorie.

5-(N,N-Dimethyl-carbonyl)-2,4,6-trijod-isophthalsäure: Fp. oberhalb 300°C (Zersetzung); Ausbeute 72% der Theorie.

2,4,6-Trijodbenzol-1,3,5-tricarbonsäure-mono-methylamid: Fp. oberhalb 300°C (Zersetzung); Ausbeute 79% der Theorie.

Für die erfindungsgemäss durchgeführten Amidierungsreaktionen geht man zweckmässigerweise von Vorprodukten bzw. Ausgangsprodukten aus, in denen die zu amidierende Carboxylgruppe als Säurehalogenid, z.B. Säurechlorid, vorliegt. Die Überführung der Carboxylgruppe in die Säurehalogenidgruppe erfolgt nach Methoden, wie sie dem Fachmann allgemein geläufig sind und am Beispiel 2,4,6-Trijod-trimesinsäure-trichlorid-Herstellung gezeigt wird:

147 g 2,4,6-Trijod-trimesinsäure, 588 ml Thionylchlorid und 1,3 ml Dimethylformamid werden 2 Stunden unter Rückfluss auf dem Dampfbad gerührt, wobei anfangs heftige HCl-Entwicklung eintritt. Die Lösung wird dann im Vakuum bei ca. + 50°C eingeengt und der Rückstand 2 Stunden mit 1,5 Liter Toluol gerührt. Wenig ungelöste Substanz wird abgesaugt und verworfen. Das Filtrat wird im Vakuum bei ca. + 50°C eingeengt und der Rückstand bei + 60°C im Vakuum getrocknet: Ausbeute 151 g (93,9% der Theorie), Fp. 258-260°C; Ausbeute 93,3%.

In analoger Weise erhält man z.B. aus den entsprechenden Säuren:

5-Cyano-2,4,6-trijod-isophthalsäure-dichlorid: Fp. 278-280°C (aus Toluol); Ausbeute 90% der Theorie.

5-Carbamoyl-2,4,6-trijod-isophthalsäure-dichlorid: Fp. 247-248°C (Zersetzung); Ausbeute 58,7% der Theorie.

5-N-Methylcarbamoyl-2,4,6-trijod-isophthalsäure-dichlorid: Fp. 214-216°C; Ausbeute 97,9% der Theorie.

5-N,N-Dimethyl-carbamoyl-2,4,6-trijod-isophthalsäure-dichlorid: Fp. 272-273°C; Ausbeute 85% der Theorie.

2,4,6-Trijodbenzol-1,3,5-tricarbonsäure-dichlorid-mono-(2-hydroxyäthyl)-amid: Fp. 75-85°C; Ausbeute 93% der Theorie.

2,4,6-Trijodbenzol-1,3,5-tricarbonsäure-monochlorid-bis-(2-acetoxyäthyl)-diamid: Fp. 202 bis 204°C; Ausbeute 75% der Theorie.

2,4,6-Trijod-3,5-bis-(N-methylcarbamoyl)-benzoesäurechlorid: Fp. 293-295°C (Zersetzung); Ausbeute 96% der Theorie.

Nach dem erfindungsgemässen Verfahren ist es auch möglich, von Vor- bzw. Ausgangsprodukten auszugehen, in denen der letztlich gewünschte Amidrest -N-$R_1 \cdot R_2$ bereits enthalten ist. Die Einführung dieses Amidrestes erfolgt dann in geeignete Vorstufen nach Methoden, wie sie dafür dem Fachmann allgemein geläufig sind und vorzugsweise nach Methoden, wie sie bereits oben, insbesondere mit den Erläuterungen zur Verfahrensvariante a, behandelt wurden. In diesem Zusammenhang sei die Einführung des Amidrestes nochmals am Beispiel der Herstellung von 5-Cyano-2,4-6-trijod-isophthalsäure-bis(2,3-dihydroxypropyl-N-methyl)-diamid aus dem Dichlorid dargestellt:

100 g 5-Cyano-2,4,6-trijod-isophthalsäuredichlorid werden in 200ml Dimethylacetamid bei 50°C gelöst. Hierzu wird eine Lösung von 42,8 g 1-N-Methylamino-propandiol-(2,3) in 120 ml Dimethylacetamid innerhalb 10 Minuten zugetropft. Nach Zugabe von 97 ml n-Tributylamin wird das Reaktionsgemisch 4 Stunden bei 50°C gerührt. Nach Abkühlen über Nacht auf Raumtemperatur setzt man 13,5 ml konz. Salzsäure zu und giesst in 7 l Methylenchlorid ein.

Nach einstündigem Rühren wird der Niederschlag abgesaugt und mehrfach mit Methylenchlorid gewaschen. Nach dem Lösen in 750 ml Wasser wird abdestilliert und die wässrige Lösung mit einem Kationen- und einem Anionenaustauscherharz behandelt. Dann wird die filtrierte neutrale wässrige Lösung im Vakuum konzentriert, über Aktivkohle filtriert und zur Trockne im Vakuum eingeengt. Man erhält 86 g (70% der Theorie) 5-Cyano-2,4,6-trijod-isophthalsäure-bis(2,3-dihydroxypropyl-N-methyl)-diamid als weisses Pulver mit einem Zersetzungspunkt oberhalb 280°C.

In analoger Weise erhält man:

5-Cyano-2,4,6-trijod-isophthalsäure-bis[bis(2--hydroxyäthyl)]-diamid: Fp. 212-215°C; Ausbeute 78% der Theorie.

5-Cyano-2,4,6-trijod-isophthalsäure-bis[tris(hydroxy-methyl)-methyl]-diamid: Fp. oberhalb 280°C; Ausbeute 70% der Theorie.

5-Cyano-3-(2,3-dihydroxypropylcarbamoyl)--2,4,6-trijodbenzoylchlorid: Fp. 285-288°C; Ausbeute 60% der Theorie.

5-Cyano-2,4,6-trijod-isophthalsäure-bis-(2,3-dihydroxypropyl)-diamid: Fp. oberhalb 280°C; Ausbeute 80% der Theorie.

5-Cyano-2,4,6-trijod-isophthalsäure-mono-(2,3--dihydroxypropyl)-mono-(2,3-dihydroxypropyl-N--methyl)-diamid: Fp. 215°C; Ausbeute 81% der Theorie.

Wie bereits oben ausgeführt, können primäre oder sekundäre Amidgruppen in an sich bekannter Weise N-alkyliert werden. Häufig ist es zweckmässig (z.B. um einen gegebenenfalls notwendigen intermediären Schutz von im Molekül anwesenden Hydroxylgruppen zu vermeiden) von N-$R_1$-substituierten Vor- oder Ausgangsprodukten auszugehen. Auch in diesen Fällen erfolgt die Einführung des Alkylrestes $R_1$ nach den an sich bekannten Methoden, wie am Beispiel der Herstellung von 5-N,N-Dimethyl-carbamoyl-2,4,6-trijodisophthalsäure aus der entsprechenden 5-Carbamoyl-Verbindung nochmals im einzelnen dargestellt werden soll:

200 g 5-Carbamoyl-2,4,6-trijod-isophthalsäure werden in 830 ml 8n-Natronlauge gelöst. Dann setzt man eine Lösung von 400 ml Dimethylsulfat in 400 ml Aceton zu und erwärmt 6 Stunden auf 60°C. Nach Aküȟlung über Nacht auf Raumtemperatur gibt man 1 l Essigester zu, säuert mit verdünnter Chlorwasserstoffsäure an, trennt die Essigesterphase ab und extrahiert die salzsaure Phase mit 200 ml Essigester nach. Die Essigesterphasen werden vereinigt, über Natriumsulfat getrocknet und anschliessend im Vakuum zur Trockne eingeengt. Der Rückstand wird 30 Minuten mit 750 ml Aceton am Rückfluss erhitzt. Nach mehrstündigem Rühren im Eisbad wird das ausgeschiedene Kristallisat abgesaugt, mit wenig eiskaltem Aceton gewaschen und bei 60°C getrocknet. Man erhält 188 g (90% der Theorie) 5-N,N-Dimethyl--carbamoyl-2,4,6-trijod-isophthalsäure als weisses Pulver mit einem Zersetzungspunkt oberhalb 280°C.

Zur Herstellung von erfindungsgemässen Verbindungen der Formel I mit X in der Bedeutung -$CH_2OH$ ist es zweckmässig von Vor- bzw. Ausgangsprodukten auszugehen, in denen der Rest -$CH_2OH$ bereits vorgegeben ist. Vorteilhafterweise geht man z.B. aus von der bekannten 5-Amino-3-hydroxymethyl--2,4,6-trijodbenzoesäure und substituiert die Aminogruppe durch die Cyanogruppe, wie bereits oben dargestellt und im nachfolgenden Beispiel nochmals im einzelnen erläutert werden soll:

171 g 5-Amino-3-hydroxymethyl-2,4,6-trijodbenzoesäure werden in 3 l Wasser suspendiert und durch Zugabe von verdünnter Natronlauge in Lösung gebracht. Dann stellt man durch Zugabe von halbkonzentrierter Schwefelsäure einen $p_H$-Wert von 2,5 ein und tropft unter Kühlung innerhalb von 20 Minuten eine Lösung von 28 g Natriumnitrit in 96 ml Wasser so zu, dass die Reaktionstemperatur zwischen 0° und +5°C liegt. Man stellt danach den $p_H$-Wert mit halbkonzentrierter Schwefelsäure erneut auf 2,5 ein und rührt 2 Stunden im Eisbad nach. Anschliessend neutralisiert man das Reaktionsgemisch unter Kühlen mit verdünnter Natronlauge und giesst in eine Lösung von 143 g Kupfer-I-cyanid und 267 g Kaliumcyanid in 1,3 l Wasser ein, wobei starkes Schäumen eintritt. Man lässt 30 Minuten bei Raumtemperatur nachrühren, bringt durch Zugabe von konzentrierter Salzsäure auf $p_H$ 2,8, saugt die ausgeschiedenen Kupfersalze ab, bringt das Filtrat durch weitere Salzsäurezugabe auf $p_H$ 0,5, lässt mehrere Stunden im Eisbad nachrühren und saugt dann den gebildeten Niederschlag ab. Nach Waschen mit Wasser suspendiert man ihn in 3 l Wasser, bringt durch Zugabe von konzentriertem Ammoniak weitgehend in Lösung, setzt Aktivkohle zu und lässt 30 Minuten bei Raumtemperatur rühren. Man saugt ab, versetzt das Filtrat mit überschüssiger konzentrierter Salzsäure, lässt mehrere Stunden im Eisbad nachühren, saugt den Niederschlag ab und löst ihn wasserfeucht in 1,3 l Aceton. Nach mehrstündigem Rühren im Eisbad wird das Kristallisat abgesaugt, mit eiskaltem Aceton gewaschen und bei 50°C getrocknet. Man erhält 141 g (= 81% der Theorie) 5-Cyano-3-hydroxymethyl--2,4,6-trijodbenzoesäure als weisses Pulver vom Fp. 250-252°C.

Hieraus erhält man in der oben beschriebenen Weise, z.B. durch Verseifung der CN-Gruppe zu -$CONH_2$ und Diazotierung dieser zur COOH-Gruppe, 5-Hydoxymethyl-2,4,6-trijod-isophthalsäure, die dann gemäss Verfahrensvariante c zu Estern oder in Form des Disäurechlorids gemäss Verfahrensvariante a zu Amiden weiterverarbeitet werden kann.

Gleiches gilt für die Herstellung von Verbindungen der Formel I mit X in der Bedeutung -$CH_2NH \cdot R_7$. Auch hier geht man zweckmässigerweise von Vor- bzw. Ausgangsprodukten aus, in denen der $CH_2NH \cdot R_7$-Rest vorgegeben ist. Dies sei am Beispiel 5-Cyano-3-acetylaminomethyl-2,4,6-trijodbenzoesäure näher erläutert:

14 g Natriumnitrit werden unter Rühren in 170 ml einer auf +5°C temperierten konzentrierten Schwefelsäure eingetragen. Man hält anschliessend so lange bei +70°C, bis Lösung eingetreten ist und kühlt dann auf +5°C ab. Nach Zutropfen von 84 ml Eisessig unter Kühlung trägt man portionsweise 93,6 g 5-Amino-3-acetylaminomethyl-2,4,6-trijodbenzoesäure ein, wobei die Reaktionstemperatur zwischen 0° und +5°C gehalten wird. Man lässt das Reaktionsgemisch 2 Stunden nachrühren, giesst auf 800

g Eis und trägt unter Kühlung in eine Lösung von 71 g Kupfer-I-cyanid und 133 g Kaliumcyanid in einer Mischung aus 1 l konzentriertem Ammoniak und 640 ml Wasser ein. Nach mehrstündigem Rühren im Eisbad wird das ausgeschiedene Ammoniumsalz abgesaugt und in 4 l Wasser gelöst. Nach Filtration über Aktivkohle stellt man das Filtrat mit Essigsäure auf $p_H$ 6 ein, rührt erneut mit Aktivkohle aus, filtriert und versetzt das Filtrat mit überschüssiger konzentrierter Salzsäure und trocknet den abgetrennten Niederschlag bei 50°C. Man erhält 80,5 g (= 85% der Theorie) 5-Cyano-3-acetyl-aminomethyl-2,4,6-trijodbenzoesäure als weisses Pulver vom Fp. 271°C (Z).

Soll dieses Vorprodukt in ein Ausgangsprodukt der Formel II weiterverarbeitet werden, wird, wie oben beschrieben, z.B. die Cyanogruppe zunächst zur Amidgruppe $-CO \cdot NH_2$ verseift und diese mittels eines Diazotierungsmittels (vorzugsweise mittels $NaNO_2$ in wässrig-saurer Lösung) in die Carboxylgruppe überführt. Die so erhaltene 5-ständige $CH_2 \cdot NH \cdot R_7$-substituierte 2,4,6-Trijodisophthalsäure, z.B. 5-Acetylaminomethyl-2,4,6-trijodisophthalsäure, wird dann gemäss Verfahrensvariante c oder in Form seines z.B. Disäurechlorids gemäss Verfahrensvariante a in die letztlich gewünschte erfindungsgemässe Verbindung der Formel I weiterverarbeitet.

Sollen Endprodukte der allgemeinen Formel I hergestellt werden, in denen Z den Rest $-NH \cdot R_7$ bedeutet, ist es gegebenenfalls zweckmässig, den 5-ständigen Substituenten $-CONH \cdot R_7$ schon in einer früheren Vorstufe einzuführen. Bevorzugt geeignet sind Vorstufen mit einer 5-ständigen Cyanogruppe, an deren CN-Dreifach-Bindung man in an sich bekannter Weise — und wie oben bei Darstellung der Verfahrensvariante b erläutert — die letztlich gewünschte aliphatische Carbonsäure $R_7COOH$ anlagert. Am Beispiel des 5-Cyano-2,4,6-trijodisophthalsäure-mono-dimethylamids sei die $R_7COOH$-Anlagerung nochmals im einzelnen erläutert:

6 g 5-Cyano-2,4,6-trijod-isophthalsäure-mono-dimethylamid werden in 30 ml Essigsäureanhydrid suspendiert. Nach Zugabe von 0,5 ml 80%iger Perchlorsäure hält man das Reaktionsgemisch 3 Stunden bei 90-95°C, filtriert über Aktivkohle, tropft unter Kühlung in 200 ml Wasser ein und bringt durch Zugabe von Natriumcarbonat bei $p_H$ 10 in Lösung. Nach Behandeln mit Aktivkohle wird die Lösung mit überschüssiger konzentrierter Salzsäure versetzt und der erhaltene Niederschlag nach Absaugen und Waschen mit Wasser in 40 ml Aceton gelöst. Nach mehrstündigem Rühren im Eisbad wird das Kristallisat abgesaugt, mit wenig eiskaltem Aceton gewaschen und bei 50°C getrocknet. Man erhält 4,5 g 5-Acetylaminocarbonyl-2,4,6-trijod-isophthalsäure-mono-dimethylamid als weisses Pulver mit einem oberhalb 280°C liegenden Zersetzungspunkt.

In analoger Weise erhält man aus der entsprechenden 5-Cyano-Verbindung:

5-Acetylaminocarbonyl-2,4,6-trijodisophthalsäure: Fp. 185°C (Zersetzung); Ausbeute 80% der Theorie.

Ist es im Verlauf des Verfahrens zur Herstellung der erfindungsgemässen Verbindungen der Formel I notwendig, eine intermediär entstehende Säurehalogenid-, z.B. Säurechlorid-Gruppe zur freien Carboxylgruppe zu verseifen, so erfolgt auch diese Verseifungsreaktion nach allgemein gebräuchlichen Methoden, wie am Beispiel 5-Chloroformyl-2,4,6-trijodisophthalsäure-[(2,3-dihydroxypropyl)-(N,N-dimethyl)]-diamid im einzelnen dargestellt werden soll:

7 g 5-Chloroformyl-2,4,6-trijod-isophthalsäure-[(2,3-dihydroxypropyl)-(N,N-dimethyl)]-diamid werden in 15 ml Dimethylsulfoxid gelöst. Nach Zugabe von 5 ml 2n-Natronlauge lässt man das Gemisch über Nacht bei Raumtemperatur stehen und engt dann im Vakuum zur Trockne ein. Der Rückstand wird in 20 ml Wasser aufgenommen, über Aktivkohle filtriert und unter Rühren mit überschüssiger konzentrierter Salzsäure versetzt. Nach mehrstündigem Rühren wird der gebildete Niederschlag abgesaugt, mit wenig eiskaltem Wasser gewaschen und bei 50°C getrocknet. Man erhält 6,2 g (= 90% der Theorie) 5-N,N-Dimethylcarbamoyl-2,4,6-trijodisophthalsäure-mono-2,3-dihydroxypropylamid als weisses Pulver vom Fp. 255-258°C (Z).

Wie bereits oben ausgeführt, erfolgt die Veresterung freier Carboxylgruppen nach an sich bekannten Methoden, wobei Methoden, wie sie für die Veresterung von trijodierten Aminobenzoesäure-Derivaten beschrieben sind, bevorzugt sind und wie mit nachfolgendem Beispiel nochmals erläutert wird:

60 g 5-Cyano-2,4,6-trijod-isophthalsäure-monomethyl-amid werden in 150 ml Dimethylformamid mit 22 g Natriumcarbonat (wasserfrei) und 28 g 1-Chlor-propandiol (2,3) 4 Stunden auf 90°C erhitzt. Man kühlt dann ab, saugt vom ausgeschiedenen Kochsalz ab und engt im Vakuum zur Trockne ein. Der Rückstand wird in 250 ml Essigester gelöst, die Lösung über Aktivkohle filtriert und das Filtrat auf die Hälfte eingeengt. Man kühlt im Eisbad ab und versetzt vorsichtig mit so viel Diisopropyläther, dass Kristallisation einsetzt. Nach mehrstündigem Nachrühren im Eisbad wird das Kristallisat abgesaugt, mit Diisopropyläther gewaschen und bei 50°C getrocknet. Man erhält 48,5 g (= 74% der Theorie) 5-Cyano-3-methylcarbamoyl-2,4,6-trijodbenzoesäure-(2,3-dihydroxypropyl)ester als weisses Pulver vom Fp. 117 bis 120°C.

Verfahrensgemässe Ausgangsprodukte lassen sich auch herstellen aus 3,5-Diamino-2,4,6-trijod-benzoesäure, indem man in analoger Weise wie oben beschrieben die 3- und 5-ständige Aminogruppe durch die Cyanogruppe substituiert und diese anschliessend zur Carbamoylgruppe verseift. Die so erhaltene 3,5-Bis-carbamoyl-2,4,6-trijodbenzoesäure wird dann wie oben beschrieben zur 2,4,6-Trijodtrimesinsäure diazotiert.

7 g Natriumnitrit werden unter Rühren in 84 ml einer auf +5°C temperierten konz. Schwefelsäure eingetragen. Man hält anschliessend so lange bei +70°C, bis Lösung eingetreten ist und kühlt dann auf +5°C ab. Nach Zutropfen von 42 ml Eisessig unter gutem Kühlen trägt man portionsweise 21 g 3,5-Diamino-2,4,6-trijodbenzoesäure (über das Dioxanaddukt gereinigt) unter Rühren so ein, dass die Innentemperatur zwischen 0° und +5°C liegt. Man lässt den Ansatz noch 2 Stunden nachrühren und giesst die grüngefärbte Suspension auf 400 g Eis

auf. Man versetzt 500 ml konz. Ammoniak mit 320 ml Wasser und löst darin 35,6 g Kupfer-I-cyanid und 67 g Kalimcyanid. Zu dieser Lösung gibt man den Diazotierungsansatz, wobei starkes Schäumen eintritt. Man lässt noch2 Stunden nachrühren und versetzt den Ansatz nach Stehen über Nacht zuerst mit 500 ml Essigester, dann mit überschüssiger konz. Salzsäure. Nach Absaugen der ausgeschiedenen Kupfersalze, die mit Essigester ausgewaschen werden, trennt man im Filtrat die wässrige Phase ab und extrahiert sie mehrfach mit Essigester nach. Die Essigesterextrakte werden vereinigt, mit Wasser zurückgewaschen und anschliessend über Natriumsulfat getrocknet und eingeengt. Der dunkelgefärbte Rückstand wird heiss mit 100 ml Aceton behandelt, die acetonische Lösung von ungelösten Anteilen filtriert und anschliessend auf die Hälfte eingeengt. Nach mehrstündigem Rühren wird das Kristallisat abgesaugt, mit eiskaltem Aceton gewaschen und bei 50°C getrocknet. Man erhält 8,5 g (= 38% der Theorie) 3,5-Dicyano-2,4,6-trijodbenzoesäure als weisses Pulver mit einem über 280°C liegenden Zersetzungspunkt.

10 g 3,5-Dicyano-2,4,6-trijodbenzoesäure werden in 100 ml Wasser suspendiert und durch Zugabe von 2 g Natriumhydroxid in Lösung gebracht. Das Reaktionsgemisch wird 3 Stunden auf +60°C gehalten. Anschliessend wird die so erhaltene 3,5-Biscarbamoyl-2,4,6-trijodbenzoesäure-Lösung mit 100 ml konz. Salzsäure versetzt und auf 90°C erwärmt. Unter die Oberfläche der Lösung wird unter ständigem Rühren innerhalb von 5 Stunden eine Lösung von 12 g Natriumnitrit in 60 ml Wasser zugeleitet. Man lässt noch 2 Stunden bei 90°C nachrühren, engt dann im Vakuum zur Trockne ein und rührt den Rückstand mit 100 ml Diisopropyläther aus. Man saugt vom ausgeschiedenen Natriumchlorid ab und engt das Filtrat zur Trockne ein. Man erhält 10 g 2,4,6-Trijodbenzol-1,3,5-tricarbonsäure als weisses Pulver mit einem oberhalb 280°C liegenden Zersetzungspunkt; Ausbeute 93% der Theorie.

Gewünschtenfalls kann aus der oben erhaltenen 3,5-Biscarbamoyl-2,4,6-trijodbenzoesäure-Natriumsalz-Lösung die 3,5-Biscarbamoyl-2,4,6-trijodbenzoesäure in üblicher Weise isoliert werden: Fp. oberhalb 300°C; Ausbeute 90%.

*Beispiel 1*

*2,4,6-Trijodbenzol-1,3,5-tricarbonsäure-tris-(2,3-dihydroxy-propyl-N-methyl)-triamid*

127,1 g Trijodtrimesinsäure-trichlorid werden in 254 ml Dimethylacetamid bei 50°C gelöst. Dann tropft man innerhalb 15 Minuten unter Rühren eine Lösung von 145,2 g N-Methylaminopropandiol (2,3) so zu, dass eine Innentemperatur von 60°C nicht überschritten wird. Anschliessend wird das Reaktionsgemisch 4 Stunden bei 50°C gerührt. Nach Stehen über Nacht setzt man 20 ml konz. Chlorwasserstoffsäure zu und engt im Vakuum zur Trockne ein. Der Rückstand wird in 1 l Wasser gelöst und zuerst mit 1,5 l eines Kationenaustauscherharzes, darauf das Filtrat mit 1,5 l eines Anionenaustauscherharzes behandelt. Dann engt man das farblose salzfreie Filtrat im Vakuum zur Trockne ein und trocknet den Rückstand bei 50°C.

Fp. 150-152°C; Ausbeute: 134 g (80% der Theorie).

*Beispiel 2*

*2,4,6-Trijodbenzol-1,3,5-tricarbonsäure-tris-(2,3,4,5,6-pentahydroxyhexyl-N-methyl)-triamid*

68,3 g N-Methylglucamin werden in 175 ml Dimethylacetamid suspendiert, die Suspension wird anschliessend auf 50°C erwärmt. Dann tropft man unter Rühren bei 50°C eine Lösung von 32,2 g Trijodtrimesinsäure-trichlorid in 75 ml Dimethylacetamid innerhalb 15 Minuten zu. Anschliessend wird das Reaktionsgemisch noch 4 Stunden bei 50°C gerührt. Nach Stehen über Nacht setzt man 6 ml konz. Chlorwasserstoffsäure zu und lässt 30 Minuten nachrühren. Man saugt das ausgeschiedene N-Methylglucamin-Hydrochlorid ab, das nach Waschen mit wenig Dimethylacetamid verworfen wird. Das Filtrat wird im Vakuum eingeengt und das zurückbleibende Rohprodukt analog Beispiel 1 über Austauschharze gereinigt.

Fp. 112-119°C; Ausbeute: 34,3 g (61% der Theorie).

*Beispiel 3*

*2,4,6-Trijodbenzol-1,3,5-tricarbonsäure-tris-(bis-2-hydroxyäthyl)-triamid*

113 g Trijodtrimesinsäure-trichlorid werden in 226 ml Dimethylacetamid bei 50°C gelöst. Dann tropft man innerhalb 25 Minuten eine Lösung von 73,9 g Diäthanolamin in 148 ml Dimethylacetamid so zu, dass eine Innentemperatur von 60°C nicht überschritten wird. Anschliessend wird das Reaktionsgemisch 4 Std. bei 50°C gerührt. Nach Stehen über Nacht setzt man 30 ml Chlorwasserstoffsäure zu und tropft die Lösung in 2,8 l Methylenchlorid unter Rühren ein. Man lässt noch 1 Stunde nachrühren, dekantiert das überstehende Methylenchlorid ab und rührt den Rückstand erneut mit 1 l Methylenchlorid aus. Der abgetrennte Rückstand wird im Vakuum bei 50°C getrocknet, dann in 1 l Wasser gelöst und die Lösung analog Beispiel 1 durch Behandeln mit Austauscherharzen gereinigt. Das gewünschte Produkt wird durch Einengen der wässrigen Lösung im Vakuum isoliert.

Fp. 132-135°C; Ausbeute: 72,3 g (49% der Theorie).

*Beispiel 4*

*2,4,6-Trijodbenzol-1,3,5-tricarbonsäure-tris-[1,1-bis-(hydroxymethyl)-methyl]-triamid*

wird analog Beispiel 3 mit 2-Aminopropandiol(1,3) erhalten.

Fp. > 300°C; Ausbeute: 68% der Theorie.

*Beispiel 5*

*2,4,6-Trijodbenzol-1,3,5-tricarbonsäure-tris-(2,3-dihydroxypropyl)-triamid*

wird analog Beispiel 3 mit 1-Aminopropandiol(1,3) erhalten.

Fp. > 300°C; Ausbeute: 70% der Theorie.

*Beispiel 6*

*(—)2,4,6-Trijodbenzol-1,3,5-tricarbonsäure-tris-[(R)-2,3-dihydroxypropyl]-triamid*

wird analog Beispiel 3 mit (+)(R)-1-Aminopropandiol(2,3) als Base erhalten.

Fp. > 300°C; $[\alpha]_D^{20}$ = 3,5°; Ausbeute: 66% der Theorie.

*Beispiel 7*

*2,4,6-Trijodbenzol-1,3,5-tricarbonsäure-tris-(2-hydroxyäthyl)-triamid*

12,9 g 2,4,6-Trijodtrimesinsäure-trichlorid werden in 26 ml Dimethylacetamid gelöst. Dann tropft man innerhalb 10 Minuten eine Lösung von 8,4 ml Äthanolamin in 20 ml Dimethylacetamid so zu, dass eine Innentemperatur von 60°C nicht überschritten wird. Anschliessend wird das Reaktionsgemisch 4 Stunden bei 50°C gerührt. Nach Stehen über Nacht werden 2 ml konz. Chlorwasserstoffsäure zugegeben und die Lösung im Vakuum eingeengt. Der Rückstand wird mit 20ml Wasser verrührt, abgesaugt und bei 50°C getrocknet.

Fp. > 300°C; Ausbeute: 13,0 g (91% der Theorie).

*Beispiel 8*

*2,4,6-Trijodbenzol-1,3,5-tricarbonsäure-tris-(2-hydroxyäthyl-N-methyl)-triamid*

wird analog Beispiel 7 mit N-Methyl-äthanolamin als Base erhalten.

Fp. 284-285°C; Ausbeute: 60% der Theorie.

*Beispiel 9*

*2,4,6-Trijodbenzol-1,3,5-tricarbonsäure-tris-(2-hydroxypropyl)-triamid*

wird analog Beispiel 7 mit 1-Aminopropanol(2) als Base erhalten.

Fp. 294-295°C; Ausbeute: 65% der Theorie.

*Beispiel 10*

*5-Carbamoyl-2,4,6-trijod-isophthalsäure-bis-(2,3-dihydroxypropyl-N-methyl)-diamid*

200 g 5-Cyano-2,4,6-trijod-isophthalsäure-bis-(2,3-dihydroxypropyl-N-methyl)-diamid werden in 540 ml Wasser und 69,5 ml konz. Natronlauge gelöst und 3 Stunden bei 60°C gerührt. Die Lösung wird analog Beispiel 3 mit konz. Chlorwasserstoffsäure versetzt, die Lösung über Ionenaustauscher gereinigt und das Produkt durch Einengen der salzfreien wässrigen Lösung im Vakuum bei 50°C erhalten.

Fp. 192-194°C; Ausbeute: 147 g (72% der Theorie).

*Beispiel 11*

*5-Carbamoyl-2,4,6-trijodisophthalsäure-N,N,N',N'-tetrakis-(2-hydroxyäthyl)-diamid*

wird analog Beispiel 10 durch partielle Verseifung von 5-Cyano-2,4,6-trijodisophthalsäure-N,N,N',N'-tetrakis-(2-hydroxyäthyl)-diamid erhalten.

Fp. 198-200°C; Ausbeute: 69% der Theorie.

*Beispiel 12*

*5-Carbamoyl-2,4,6-trijodisophthalsäure-bis-[tris-(hydroxymethyl)-methyl]-diamid*

wird analog Beispiel 10 durch partielle Verseifung von 5-Cyano-2,4,6-trijodisophthalsäure-bis-[tris-(hydroxymethyl)-methyl]-diamid erhalten.

Fp. > 300°C; Ausbeute: 67% der Theorie.

*Beispiel 13*

*5-Carbamoyl-2,4,6-trijodisophthalsäure-bis-(2,3-dihydroxypropyl)-diamid*

95 g 5-Cyano-2,4,6-trijodisophthalsäure-bis-(2,3-dihydroxypropyl)-diamid werden unter Rühren in eine Lösung von 20 g Natriumhydroxid in 500 ml Wasser portionsweise eingetragen. Man lässt 4 Stunden bei Raumtemperatur rühren. Die klare Lösung wird mit konz. Chlorwasserstoffsäure auf $p_H$ 7 gebracht, über Aktivkohle filtriert und im Vakuum eingeengt. Der Rückstand wird mit 500 ml eines Gemisches aus gleichen Teilen Methanol und Äthanol 30 Minuten ausgekocht. Das Natriumchlorid wird abfiltriert und das Filtrat im Vakuum zur Trockne eingeengt. Der Rückstand wird im Vakuum bei 50°C getrocknet.

Fp. > 300°C; Ausbeute: 80 g (73% der Theorie).

*Beispiel 14*

*5-Carbamoyl-2,4,6-trijodisophthalsäure-bis-[1,1-bis-(hydroxymethyl)-methyl]-diamid*

11 g 5-Carbamoyl-2,4,6-trijodisophthalsäure-dichlorid werden in 22 ml Dimethylacetamid gelöst. Dann tropft man unter Rühren innerhalb von 10 Minuten eine Lösung von 40 g 2-Aminopropandiol(1,3) in 15 ml Dimethylacetamid so zu, dass eine Innentemperatur von 60°C nicht überschritten wird. Anschliessend wird das Reaktionsgemisch 4 Stunden bei 50°C gerührt. Nach Stehen über Nacht werden 2,4 ml konz. Chlorwasserstoffsäure zugegeben und die Lösung in 270 ml Methylenchlorid unter Rühren eingetropft. Nach einer Stunde dekantiert man das überstehende Methylenchlorid ab und rührt den Rückstand erneut mit 200 ml Methylenchlorid aus. Der abgetrennte Rückstand wird im Vakuum bei 50°C getrocknet, dann in 100 ml Wasser gelöst und die über Aktivkohle filtrierte Lösung analog Beispiel 1 mit Ionenaustauscherharzen gereinigt. Das gewünschte Produkt wird durch Einengen der wässrigen Lösung im Vakuum isoliert.

Fp. 248-252°C; Ausbeute: 8,4 g (65% der Theorie).

*Beispiel 15*

*5-N-Methyl-carbamoyl-2,4,6-trijodisophthalsäure-bis-(2,3-dihydroxypropyl-N-methyl)-diamid*

62,3 g 5-N-Methyl-carbamoyl-2,4,6-trijodisophthalsäuredichlorid werden mit 125 ml auf 50°C erwärmtem Dimethylacetamid verrührt. Hierzu tropft

man unter Rühren eine Lösung von 30,8 g N-Methylaminopropandiol(2,3) in 62 ml Dimethylacetamid. Danach werden 69,6 ml Tri-n-butylamin zugegeben. Anschliessend wird das Reaktionsgemisch 4 Stunden bei 50°C gerührt. Nach Stehen über Nacht werden 18 ml konz. Chlorwasserstoffsäure zugegeben und die Lösung in 3 l Methylenchlorid eingerührt. Man lässt noch 1 Stunde nachrühren, dekantiert das überstehende Methylenchlorid ab und rührt den Rückstand erneut mit 500 ml Methylenchlorid aus. Der abgetrennte Rückstand wird im Vakuum bei 50°C getrocknet, dann in 500 ml Wasser gelöst und die über Aktivkohle filtrierte Lösung analog Beispiel 1 mit Ionenaustauscherharzen gereinigt. Das gewünschte Produkt wird durch Einengen der wässrigen Lösung im Vakuum isoliert.

Fp. 178-182°C; Ausbeute: 57,6 g (76% der Theorie).

*Beispiel 16*

*5-N-Methyl-carbamoyl-2,4,6-trijodisophthalsäure--N,N,N',N'-tetrakis-(2-hydroxyäthyl)-diamid*

wird analog Beispiel 15 mit Diäthanolamin als Base erhalten.

Fp. 167-174°C; Ausbeute: 55% der Theorie.

*Beispiel 17*

*5-N,N-Dimethyl-carbamoyl-2,4,6-trijodisophthalsäure-bis-(2,3-dihydroxypropyl)-diamid*

60 g 5-N,N-Dimethyl-carbamoyl-2,4,6-trijodisophthalsäurechlorid werden in 76 ml Dimethylacetamid bei 50°C gelöst. Dann tropft man innerhalb 10 Minuten eine Lösung von 13,9 g 1-Aminopropandiol(2,3) in 46 ml Dimethylacetamid unter Rühren so zu, dass eine Innentemperatur von 60°C nicht überschritten wird. Anschliessend wird das Reaktionsgemisch 4 Std. bei 50°C gerührt. Nach Stehen über Nacht setzt man 5 ml konz. Chlorwasserstoffsäure zu und tropft die Lösung in 2 l Methylenchlorid unter Rühren ein. Man lässt noch 1 Stunde nachrühren, dekantiert das Methylenchlorid ab, rührt den Rückstand erneut mit 500 ml Methylenchlorid aus und trocknet ihn nach Abtrennen des Methylenchlorids im Vakuum bei 50°C. Man löst in 500 ml Wasser und reinigt die Lösung alanog Beispiel 1 durch Behandeln mit Ionenaustauscherharzen. Das gewünschte Produkt wird durch Einengen der wässrigen Lösung im Vakuum isoliert.

Fp. 200-201°C; Ausbeute: 34 g (73% der Theorie).

*Beispiel 18*

*(−)-N,N-Dimethyl-carbamoyl-2,4,6-trijodisophthalsäure-bis-(2,3-dihydroxypropyl)-diamid*

wird analog Beispiel 17 mit (+)(R)-1-Aminopropandiol(2,3) erhalten.

Fp. 199-200°C; $[\alpha]_D^{20}$: −2,2°; Ausbeute: 68% der Theorie.

*Beispiel 19*

*5-N,N-Dimethyl-carbamoyl-2,4,6-trijodisophthalsäure-bis-(2,3-dihydroxypropyl-N-methyl)-diamid*

wird analog Beispiel 17 mit N-Methylaminopropandiol(2,3) als Base erhalten.

Fp. 185-187°C; Ausbeute: 69% der Theorie.

*Beispiel 20*

*5-N,N-Dimethyl-carbamoyl-2,4,6-trijodisophthalsäure-bis-[bis-(hydroxymethyl)-methyl]-diamid*

analog Beispiel 17 mit 2-Aminopropandiol(1,3) als Base.

Fp. > 300°C; Ausbeute: 72% der Theorie.

*Beispiel 21*

*5-N,N-Dimethyl-carbamoyl-2,4,6-trijodisophthalsäure-N,N,N',N'-tetrakis-(2-hydroxyäthyl)-diamid*

analog Beispiel 17 mit Diäthanolamin als Base.

Fp. 158-160°C; Ausbeute: 70% der Theorie.

*Beispiel 22*

*5-N,N-Dimethyl-carbamoyl-2,4,6-trijodisophthalsäure-bis-[tris-(hydroxymethyl)-methyl]-diamid*

analog Beispiel 17 mit 2-Amino-2-(hydroxymethyl)--propandiol(1,3) als Base.

Fp. > 300°C; Ausbeute: 67% der Theorie.

*Beispiel 23*

*5-N,N-Dimethyl-carbamoyl-2,4,6-trijodisophthalsäure-(2,3-dihydroxypropyl)-(2,3-dihydroxypropyl--N-methyl)-diamid*

5 l Dioxan werden auf 80°C erwärmt und nacheinander mit 50 g 1-Aminopropandiol(2,3) und 145 g 5-N,N-Dimethyl-carbamoyl-2,4,6-trijodisophthalsäure-dichlorid versetzt. Nach 10 Minuten Rühren bei 80°C kühlt man das Gemisch rasch ab, filtriert über Kieselgur und engt im Vakuum zur Trockne ein. Der Rückstand wird mehrfach mit je 500 ml Essigester ausgekocht, dann abgesaugt und bei 60°C getrocknet. Man erhält 69,2 g (45% der Theorie) 5-Chloroformyl-2,4,6-trijodisophthalsäure-(2,3-dihydroxypropyl)-(N,N-dimethyl)-diamid als weisses Pulver vom Fp. 145-147°C (Zersetzung).

50 g des Säurechlorids werden portionsweise unter Kühlung in eine Lösung von 15,9 g N-Methylaminopropandiol(2,3) in 144 ml Dimethylacetamid so eingerührt, dass die Reaktionstemperatur nicht über 40°C ansteigt. Nach Rühren über Nacht wird die Lösung im Vakuum eingeengt, der ölige Rückstand in 50 ml Wasser gelöst, die Lösung durch Zugabe von konz. Chlorwasserstoffsäure auf $p_H$ 7 gebracht und analog Beispiel 1 über Austauscherharze gereinigt. Das so gereinigte Produkt wird durch Einengen der wässrigen Lösung isoliert und bei 50°C getrocknet.

Fp. 105-107°C; Ausbeute: 12 g (68% der Theorie).

*Beispiel 24*

*5-Carbamoyl-2,4,6-trijodisophthalsäure-(2,3-dihydroxypropyl)-(2,3-dihydroxypropyl-N-methyl)--diamid*

wird analog Beispiel 10 durch partielle Verseifung von 5-Cyano-2,4,6-trijodisophthalsäure-(2,3-dihydroxypropyl)-(2,3-dihydroxypropyl-N-methyl)-diamid erhalten.

Fp. 200-202°C; Ausbeute: 70% der Theorie.

*Beispiel 25*

*5-N,N-Dimethyl-carbamoyl-2,4,6-trijodisophthalsäure-bis-(2,3-dihydroxypropyl)-diester*

12,2 g 5-N,N-Dimethyl-carbamoyl-2,4,6-trijodisophthalsäure werden in 100 ml Wasser suspendiert und durch Zugabe von konz. Natronlauge bei $p_H$ 7 in Lösung gebracht. Dann engt man im Vakuum zur Trockne ein und löst das wasserfreie Dinatriumsalz in 30 ml Dimethylformamid. Nach Zugabe von 11 g 1-Chlorpropandiol(2,3) wird das Reaktionsgemisch 4 Stunden auf 90°C erwärmt. Nach Abkühlen auf Raumtemperatur trennt man das Natriumchlorid ab und engt die Lösung im Vakuum ein. Der Rückstand wird mit 120 ml Aceton ausgekocht und die Lösung heiss über Aktivkohle filtriert. Man fügt 1 ml konz. Chlorwasserstoffsäure zu und engt die Lösung auf 50 ml ein. Nach Abkühlen auf 5°C versetzt man mit soviel Diisopropyläther, dass Kristallisation eintritt. Nach mehrstündigem Rühren im Eisbad wird das Kristallisat abgesaugt, mit Diisopropyläther gewaschen und bei 50°C getrocknet.

Fp. 145°C; Ausbeute: 9,4 g (62% der Theorie).

*Beispiel 26*

*5-N-Methyl-carbamoyl-2,4,6-trijodisophthalsäure--bis-(2,3-dihydroxy-1-hydroxymethyl-propyl)-diamid*

wird analog Beispiel 15 mit 2-Amino-1,3,4-trihydroxybutan als Base erhalten.

Fp. ab 185°C; Ausbeute: (58,3% der Theorie).

*Beispiel für die Darreichungsform:*

| | | |
|---|---|---|
| 5-Carbamoyl-2,4,6-trijodisophthalsäure-bis-(2,3--dihydroxypropyl-N-methyl)-diamid | | 599,74 g |
| Calciumdinatriumsalz der Äthylendiamintetraessigsäure | | 0,10 g |
| Natriumbicarbonat | | 1,23 g |
| Aqua bidestillata | ad | 100 ml |

*Ausführung:*

Das 5-Carbamoyl-2,4,6-trijodisophthalsäure-bis--(2,3-dihydroxypropyl-N-methyl)-diamid wird nach Zusatz des Calciumdinatriumsalzes der Äthylendiamintetraessigsäure in Aqua bidestillata gelöst. Der $p_H$ wird durch Zusatz von Natriumbicarbonat auf 7 gebracht, das Volumen durch Zugabe von Aqua bidestillata auf 1000 ml gebracht und die erhaltene Lösung anschliessend hitzesterilisiert.

Jodgehalt: 300 mg/ml.

**Patentansprüche**

1. Verbindungen der allgemeinen Formel I

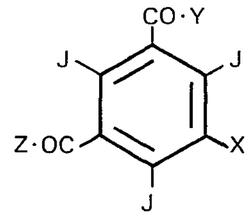

(I),

worin

X den Rest -CO·N·$R_1R_2$, -$COOR_3$, -$CH_2$·NH·$R_7$ oder -$CH_2OH$,

Y den Rest -N·$R_1R_2$ oder -$OR_3$,

Z den Rest -N·$R_1R_2$, -NR·$R_7$, -NH·Zuckerrest oder -$OR_3$, wobei -N $R_1N_2$ in X, Y und Z gleich oder verschieden sind,

$R_1$ und $R_2$ gleich oder verschieden sind und ein Wasserstoffatom oder einen gerad- oder verzweigtkettigen Alkylrest mit 1 bis 6 Kohlenstoffatomen oder einen mono- oder polyhydroxylierten gerad- oder verzweigtkettigen Alkylrest mit 2 bis 8 Kohlenstoffatomen und 1 bis 5 Hydroxylgruppen,

$R_3$ einen Alkylrest bis 1 bis 4 Kohlenstoffatomen oder einen hydroxylierten Alkylrest mit 2 bis 4 Kohlenstoffatomen und 1 bis 3 Hydroxylgruppen,

$R_7$ den Acylrest einer aliphatischen Carbonsäure mit 2 bis 5 Kohlenstoffatomen, die gegebenenfalls mit 1 bis 5 Hydroxylgruppen substituiert sein kann, wobei die Hydroxylgruppen auch funktionell abgewandelt sein können,

bedeuten.

2. 2,4,6-Trijodbenzol-1,3,5-tricarbonsäure-(2,3--dihydroxypropyl-N-methyl)-triamid.

3. 2,4,6-Trijodbenzol-1,3,5-tricarbonsäure-tris--(bis-2-hydroxyäthyl)-triamid.

4. 2,4,6-Trijodbenzol-1,3,5-tricarbonsäure-bis--(2,3-dihydroxypropyl-N-methyl)-triamid.

5. 2,4,6-Trijodbenzol-1,3,5-tricarbonsäure-tris--(2,3,4,5,6-pentahydroxyhexyl-N-methyl)-triamid.

6. 2,4,6-Trijodbenzol-1,3,5-tricarbonsäure--[(N,N-dimethyl)-bis-(2,3-dihydroxypropyl)]-triamid.

7. 5-N-Methyl-carbamoyl-2,4,6-trijodisophthalsäure-bis-(2,3-dihydroxy-1-hydroxymethyl-propyl)--diamid.

8. 5-Carbamoyl-2,4,6-trijodisophthalsäure-(2,3--dihydroxypropyl)-(2,3-dihydroxypropyl-N-methyl)--diamid.

9. Röntgenkontrastmittel enthaltend eine schattengebende Substanz gemäss Anspruch 1-8.

10. Verfahren zur Herstellung von Verbindungen der allgemeinen Formel I

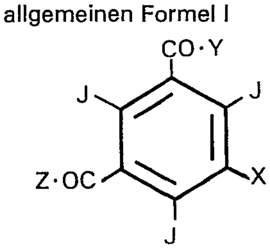

(I),

worin

X den Rest -CO·N·$R_1R_2$, -$COOR_3$, -$CH_2$·NH·$R_7$ oder -$CH_2OH$,

X den Rest $-N \cdot R_1R_2$ oder $-OR_3$,

Z den Rest $-N \cdot R_1R_2$, $-NR \cdot R_7$, -NH·Zuckerrest oder $-OR_3$, wobei $-N\ R_1R_2$ in X, Y und Z gleich oder verschieden sind,

$R_1$ und $R_2$ gleich oder verschieden sind und ein Wasserstoffatom oder einen gerad- oder verzweigtkettigen Alkylrest mit 1 bis 6 Kohlenstoffatomen oder einen mono- oder polyhydroxylierten gerad- oder verzweigtkettigen Alkylrest mit 2 bis 8 Kohlenstoffatomen und 1 bis 5 Hydroxylgruppen,

$R_3$ einen Alkylrest mit 1 bis 4 Kohlenstoffatomen oder einen hydroxylierten Alkylrest mit 2 bis 4 Kohlenstoffatomen und 1 bis 3 Hydroxylgruppen,

$R_7$ den Acylrest einer aliphatischen Carbonsäure mit 2 bis 5 Kohlenstoffatomen, die gegebenenfalls mit 1 bis 5 Hydroxylgruppen substituiert sein kann, wobei die Hydroxylgruppen auch funktionell abgewandelt sein können,

bedeuten, dadurch gekennzeichnet, dass man in an sich bekannter Weise

a) eine Verbindung der allgemeinen Formel II

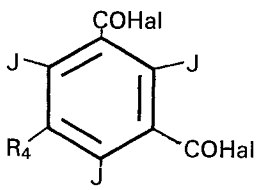

(II),

worin

Hal ein Halogenatom, vorzugsweise ein Cl-Atom, und

$R_4$ die Gruppe -COHal, $-CON<^{R_1}_{R_2}$, $-CONH-R_7$, $-CH_2 \cdot NH \cdot R_7$ oder $-CH_2OH$

(worin Hal, $R_1$, $R_2$ und $R_7$ die oben angegebene Bedeutung haben) mit einer Base der allgemeinen Formel IIIA

$$HN<^{R'_1}_{R'_2} \qquad (IIIA),$$

($R'_1$ und $R'_2$ das gleiche wie $R_1$ und $R_2$ aber nicht gleichzeitig Wasserstoff bedeuten) oder stufenweise die 1-ständige -COHal-Gruppe mit der Base IIIA und die 3-ständige -COHal-Gruppe mit einer Base IIIB der allgemeinen Formel

$$HN<^{R''_1}_{R''_2} \qquad (IIIB),$$

(worin $R''_1$ und $R''_2$ das gleiche wie $R_1$ und $R_2$ aber nicht gleichzeitig Wasserstoff bedeuten und verschieden von $R'_1$ und/oder $R'_2$ sind) umsetzt, oder

b) eine Verbindung der allgemeinen Formel IV

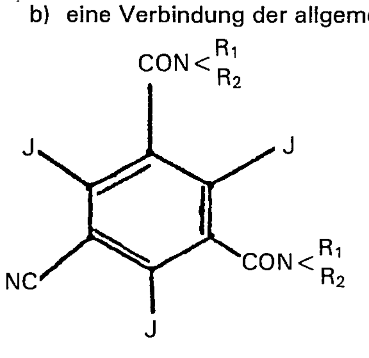

(IV),

worin $R_1$ und $R_2$ die obengenannte Bedeutung hat, mit dem reaktionsfähigen Derivat einer aliphatischen Carbonsäure $R_7$-OH umsetzt, oder in wässriger Lösung mit Alkali behandelt und die so erhaltene 5-Carbamoyl-Verbindung gewünschtenfalls anschliessend in wässriger Lösung und in Gegenwart einer starken Säure diazotiert und die gebildete 5-Carboxygruppe mit einem 2-Amino-Zucker oder mit der Base $HNR'_1R'_2$ ($R'_1$ und $R'_2$ haben die oben angegebene Bedeutung) amidiert oder mit einem Alkohol $R_3OH$ verestert oder

c) eine Verbindung der allgemeinen Formel V

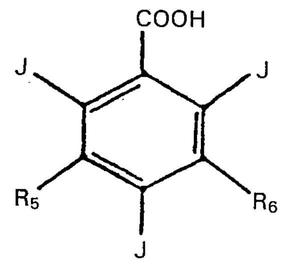

(V),

worin

$R_5$ die Reste $-CH_2OH$, $-CON<^{R^1}_{R'_2}$, $-CH_2 \cdot NH \cdot R_7$, $-CONHR_7$ oder -COOH und

$R_6$ die Reste $-CON<^{R_1}_{R_2}$ oder -COOH,

mit einem Alkohol $R_3OH$ ($R_3$ hat die oben angegebene Bedeutung) verestert,

bedeuten,

und gewünschtenfalls anschliessend Wasserstoff enthaltende Aminogruppen mit einem reaktionsfähigen Derivat einer aliphatischen Carbonsäure $R_7$-OH ($R_7$ hat die obengenannte Bedeutung) acyliert und/oder mit einem $R'_1$-enthaltenden Alkylierungsmittel N-alkyliert und/oder geschützte Hydroxylgruppen in Freiheit setzt.

**Revendications**

1. Composés répondant à la formule générale (I):

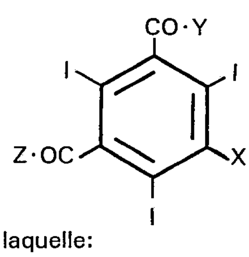

(I)

dans laquelle:

X représente un radical $-CO \cdot N \cdot R_1R_2$, $-COOR_3$, $-CH_2 \cdot NH \cdot R_7$ ou $-CH_2OH$,

Y représente un radical $-N \cdot R_1R_2$ ou $-OR_3$,

Z représente un radical $-N \cdot R_1R_2$, $-NH \cdot R_7$, -NH·(radical de sucre) ou $-OR_3$, les radicaux $-N \cdot R_1R_2$ présents dans X, Y et Z pouvant être identiques ou différents,

$R_1$ et $R_2$ sont identiques ou différents et représentent chacun un atome d'hydrogène, un radical alkyle, linéaire ou ramifié, contenant de 1 à 6 atomes de carbone, ou un radical alkyle monohydroxylé ou polyhy-

droxylé, linéaire ou ramifié, contenant de 2 à 8 atomes de carbone et portant de 1 à 5 groupes hydroxy,

$R_3$ représente un radical alkyle contenant de 1 à 4 atomes de carbone ou un radical alkyle hydroxylé contenant de 2 à 4 atomes de carbone et portant de 1 à 3 groupes hydroxy, et

$R_7$ représente le radical acyle d'un acide carboxylique aliphatique contenant de 2 à 5 atomes de carbone, radical qui peut éventuellement porter de 1 à 5 groupes hydroxy, les groupes hydroxy pouvant également se trouver sous la forme de dérivés fonctionnels.

2. Tris-[N-(dihydroxy-2,3 propyl)-N-méthyl-amide] de l'acide triiodo-2,4,6 benzène-tricarboxylique-1,3,5.

3. Tris-[bis-(hydroxy-2 éthyl)-amide] de l'acide triiodo-2,4,6 benzène-tricarboxylique-1,3,5.

4. Bis-[N-(dihydroxy-2,3 propyl)-N-méthyl]-triamide de l'acide triiodo-2,4,6 benzène-tricarboxylique-1,3,5.

5. Tris-[N-(pentahydroxy-2,3,4,5,6 hexyl)-N-méthyl-amide] de l'acide triiodo-2,4,6 benzène-tricarboxylique-1,3,5.

6. [(N,N-Diméthyl)-bis-(dihydroxy-2,3 propyl)]-triamide de l'acide triiodo-2,4,6 benzène-tricarboxylique-1,3,5.

7. Bis-[(dihydroxy-2,3 hydroxyméthyl-1 propyl)-amide] de l'acide N-méthylcarbamoyl-5 triiodo-2,4,6 isophtalique.

8. (Dihydroxy-2,3 propyl)-[N-(dihydroxy-2,3 propyl)-N-méthyl]-diamide de l'acide carbamoyl-5 triiodo-2,4,6 isophtalique.

9. Agent de contraste pour rayons X contenant un composé opacifiant selon l'une quelconque des revendications 1 à 8.

10. Procédé de préparation de composés répondant à la formule générale (I):

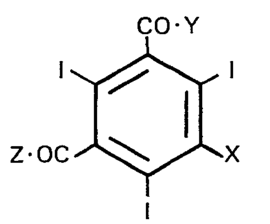

(I)

dans laquelle:

X représente un radical $-CO \cdot N \cdot R_1R_2$, $-COOR_3$, $-CH_2 \cdot NH \cdot R_7$ ou $-CH_2OH$,

Y représente un radical $-N \cdot R_1R_2$ ou $-OR_3$,

Z représente un radical $-N \cdot R_1R_2$, $-NH \cdot R_7$, $-NH \cdot$(radical de sucre) ou $-OR_3$, les radicaux $-N \cdot R_1R_2$ présents dans X, Y et Z pouvant être identiques ou différents,

$R_1$ et $R_2$ sont identiques ou différents et représentent chacun un atome d'hydrogène, un radical alkyle, linéaire ou ramifié, contenant de 1 à 6 atomes de carbone, ou un radical alkyle monohydroxylé ou polyhydroxylé, linéaire ou ramifié, contenant de 2 à 8 atomes de carbone et portant de 1 à 5 groupes hydroxy,

$R_3$ représente un radical alkyle contenant de 1 à 4 atomes de carbone ou un radical alkyle hydroxylé contenant de 2 à 4 atomes de carbone et portant de 1 à 3 groupes hydroxy, et

$R_7$ représente le radical acyle d'un acide carboxylique aliphatique contenant de 2 à 5 atomes de carbone, radical qui peut éventuellement porter de 1 à 5 groupes hydroxy, les groupes hydroxy pouvant également se trouver sous la forme de dérivés fonctionnels,

procédé caractérisé en ce que, en opérant de manière connue:

a) on fait réagir un composé répondant à la formule générale (II):

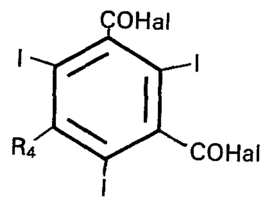

(II)

dans laquelle Hal représente un atome d'halogène, de préference de chlore, et $R_4$ un radical -COHal, $-CON<^{R^1}_{R_2}$, $-CONH-R_7$, $-CH_2 \cdot NH \cdot R_7$ ou $-CH_2OH$ (où Hal, $R_1$, $R_2$ et $R_7$ ont les significations indiquées ci-dessus), avec une base répondant à la formule générale (IIIA):

$$NH<^{R'_1}_{R'_2} \qquad (IIIA)$$

dans laquelle $R'_1$ et $R'_2$ ont les mêmes significations que $R_1$ et $R_2$ et ne peuvent représenter chacun en même temps l'hydrogène, ou on fait réagir par étapes le radical halogénocarbonyle en 1 avec la base (IIIA) et le radical halogénocarbonyle en 3 avec une base répondant à la formule générale (IIIB):

$$NH<^{R'_1}_{R'_2} \qquad (IIIB)$$

dans laquelle $R''_1$ et $R''_2$ ont les mêmes significations que $R_1$ et $R_2$ mais ne peuvent pas représenter chacun en même temps l'hydrogène et forment une paire différente de la paire formée par $R'_1$ et $R'_2$, ou

b) on fait réagir un composé répondant à la formule générale (IV):

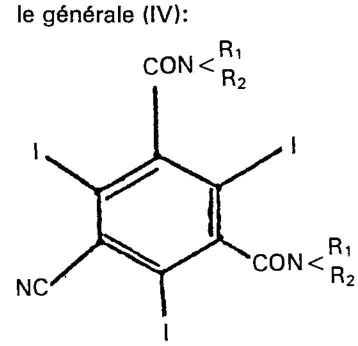

(IV)

dans laquelle $R_1$ et $R_2$ ont les significations précédemment données, avec un dérivé réactif d'un acide carboxylique aliphatique $R_7$-OH, ou on le traite en solution aqueuse par un alcali, puis, si on le désire, on diazote le composé carbamoylique-5 ainsi obtenu, en solution aqueuse et en présence d'un acide fort, et on amide le radical carboxy-5 formé avec un sucre aminé en 2 ou avec la base $HNR'_1R'_2$ ($R'_1$ et $R'_2$ ont

les significations indiquées ci-dessus) ou on l'estérifie avec un alcool $R_3OH$, ou

c) on estérifie avec un alcool $R_3OH$ (dans lequel $R_3$ a la signification indiquée plus haut) un composé répondant à la formule générale (V):

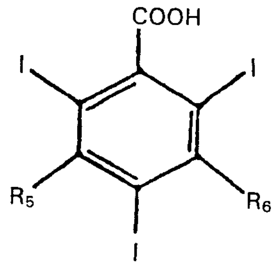

(V)

dans laquelle $R_5$ représente un radical $-CH_2OH$, $-CON{<}^{R_1}_{R_2}$, $-CH_2 \cdot NH \cdot R_7$, $-CONHR_7$ ou $-COOH$ et $R_6$ représente un radical $-CON{<}^{R_1}_{R_2}$ ou $-COOH$,

après quoi, si on le désire, on acyle les groupes amino contenant de l'hydrogène avec un dérivé réactif d'un acide carboxylique aliphatique $R_7$-OH (dans lequel $R_7$ a la signification donnée plus haut) et/ou on les alkyle à l'azote avec un agent d'alkylation contenant $R'_1$ et/ou on libère des groupes hydroxy protégés.

**Claims**

1. Compounds of the general formula I

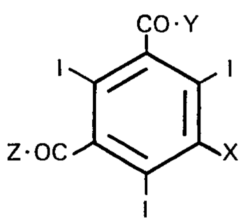

(I),

in which

X represents the radical $-CO \cdot N \cdot R_1R_2$, $-CCOR_3$, $-CH_2 \cdot NH \cdot R_7$ or $-CH_2OH$,

Y represents the radical $-N \cdot R_1R_2$ or $-OR_3$,

Z represents the radical $-N \cdot R_1R_2$, $-NH \cdot R_7$, $-NH \cdot$ (sugar radical) or $-OR_3$, $-N \cdot R_1R_2$ in X, Y and Z being the same or different,

$R_1$ and $R_2$ are the same or different and represent a hydrogen atom or a straight-chain or branched-chain alkyl radical having from 1 to 6 carbon atoms or a mono- or polyhydroxylated straight-chain or branched-chain alkyl radical having from 2 to 8 carbon atoms and from 1 to 5 hydroxy groups,

$R_3$ represents an alkyl radical having from 1 to 4 carbon atoms or a hydroxylated alkyl radical having from 2 to 4 carbon atoms and from 1 to 3 hydroxy groups, and

$R_7$ represents the acyl radical of an aliphatic carboxylic acid having from 2 to 5 carbon atoms which may optionally be substituted by from 1 to 5 hydroxy groups, it being possible for the hydroxy groups also to be functionally modified.

2. 2,4,6-Triiodobenzene-1,3,5-tricarboxylic acid tris(2,3-dihydroxypropyl-N-methyl)-triamide.

3. 2,4,6-Triiodobenzene-1,3,5-tricarboxylic acid tris(bis-2-hydroxyethyl)triamide.

4. 2,4,6-Triiodobenzene-1,3,5-tricarboxylic acid bis(2,3-dihydroxypropyl-N-methyl)triamide.

5. 2,4,6-Triiodobenzene-1,3,5-tricarboxylic acid tris(2,3,4,5,6-pentahydroxyhexyl-N-methyl)triamide.

6. 2,4,6-Triiodobenzene-1,3,5-tricarboxylic acid [(N,N-dimethyl)-bis(2,3-dihydroxypropyl)]triamide.

7. 5-N-Methylcarbamoyl-2,4,6-triiodoisophthalic acid bis(2,3-dihydroxy-1-hydroxymethylpropyl)-diamide.

8. 5-Carbamoyl-2,4,6-triiodoisophthalic acid (2,3-dihydroxypropyl)-(2,3-dihydroxypropyl-N-methyl)diamide.

9. X-ray contrast agents containing a shadow-forming substance according to claims 1 to 8.

10. Process for the preparation of compounds of the general formula I

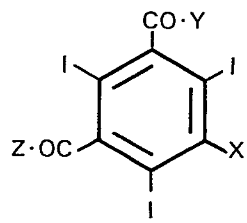

(I),

in which

X represents the radical $-CO \cdot N \cdot R_1R_2$, $-COOR_3$, $-CH_2 \cdot NH \cdot R_7$ or $-CH_2OH$,

Y represents the radical $-N \cdot R_1R_2$ or $-OR_3$,

Z represents the radical $-N \cdot R_1R_2$, $-NH \cdot R_7$, $-NH \cdot$ (sugar radical) or $-OR_3$, $-N \cdot R_1R_2$ in X, Y and Z being the same or different,

$R_1$ and $R_2$ are the same or different and represent a hydrogen atom or a straight-chain or branched-chain alkyl radical having from 1 to 6 carbon atoms or a mono- or polyhydroxylated straight-chain or branched-chain alkyl radical having from 2 to 8 carbon atoms and from 1 to 5 hydroxy groups,

$R_3$ represents an alkyl radical having from 1 to 4 carbon atoms or a hydroxylated alkyl radical having from 2 to 4 carbon atoms and from 1 to 3 hydroxy groups, and

$R_7$ represents the acyl radical of an aliphatic carboxylic acid having from 2 to 5 carbon atoms which may optionally be substituted by from 1 to 5 hydroxy groups, it being possible for the hydroxy groups also to be functionally modified,

characterised in that, in a manner known <u>per se</u>

a) a compound of the general formula II

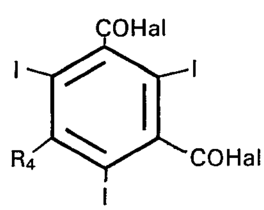

(II),

in which

Hal represents a halogen atom, preferably a chlorine atom, and $R_4$ represents the group

$-COHal$, $-CON<^{R_1}_{R_2}$, $-CONH-R_7$, $-CH_2\cdot NH\cdot R_7$ or $-CH_2-OH$
(in which Hal, $R_1$, $R_2$ and $R_7$ have the above-mentioned meanings), is reacted with a base of the general formula IIIA

$$HN<^{R'_1}_{R'_2} \quad \text{(IIIA)},$$

($R'_1$ and $R'_2$ are the same as $R_1$ and $R_2$ but do not simultaneously represent hydrogen) or, in stages, the -COHal group in the 1-position is reacted with the base IIIA and the -COHal group in the 3-position is reacted with a base IIIB of the general formula

$$HN<^{R''_1}_{R''_2} \quad \text{(IIIB)},$$

(in which $R''_1$ and $R''_2$ are the same as $R_1$ and $R_2$ but do not simultaneously represent hydrogen and are different form $R'_1$ and/or $R'_2$), or

b) a compound of the general formula IV

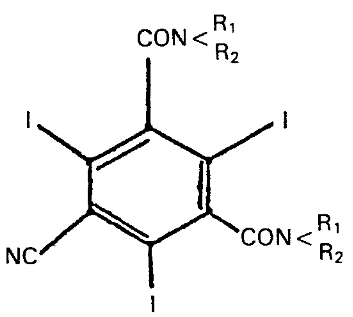

(IV),

in which $R_1$ and $R_2$ have the above-mentioned meanings, is reacted with the reactive derivative of an aliphatic carboxylic acid $R_7$-OH or treated in aqueous solution with alkali and the resulting 5-carbamoyl compound is, if desired, then diazotised in aqueous solution and in the presence of a strog acid, and the 5-carboxy group which is formed is amidated with a 2-amino sugar or with the base $HNR'_1R'_2$ ($R'_1$ and $R'_2$ have the meanings given above) or esterified with an alcohol $R_3OH$, or

c) a compound of the general formula V

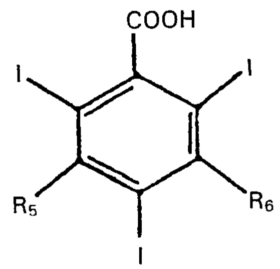

(V),

in which
$R_5$ represents the radicals $-CH_2OH$, $-CON<^{R_1}_{R_2}$, $-CH_2\cdot NH\cdot R_7$, $-CONHR_7$ or -COOH and
$R_6$ represents the radicals $-CON<^{R_1}_{R_2}$ or -COOH,

is esterified with an alcohol $R_3OH$ ($R_3$ has the meaning given above), and, if desired, amino groups containing hydrogen are then acylated with a reactive derivative of an aliphatic carboxylic acid $R_7$-OH ($R_7$ has the above-mentioned meaning) and/or N-alkylated with an alkylating agent containing $R'_1$ and/or protected hydroxy groups are freed.